# EUROPEAN PATENT SPECIFICATION

(11) **EP 3 135 225 B1**
(45) Date of publication and mention of the grant of the patent: **14.08.2019**
(21) Application number: 16187137.1
(22) Date of filing: 12.03.2014
(51) Int. Cl.: A61B 17/072, A61B 17/28, A61B 17/00, A61B 17/10, A61B 17/29, A61B 90/00

(54) **SURGICAL STAPLING APPARATUS**
CHIRURGISCHE KLAMMERVORRICHTUNG
APPAREIL D'AGRAFAGE CHIRURGICAL

(30) Priority: 13.03.2013 US 201361779873 P; 21.06.2013 US 201313923651
(43) Date of publication of application: 01.03.2017
(62) Divisional of application: 14159099.2
(73) Proprietor: Covidien LP, Mansfield, MA 02048 (US)
(72) Inventor: KOSTRZEWSKI, Stanislaw, Newtown, CT 06470 (US); WILLIAMS, Justin, Southbury, CT 06488 (US); ARANYI, Ernest, Easton, CT 06612 (US); OLSON, Lee Ann, Wallingford, CT 06492 (US); CAPPOLA, Kenneth M., Monroe, CT 06468 (US); MARCZYK, Stanislaw, Stratford, CT 06614 (US); HESSLER, Thomas R., BETHEL, CT 06801 (US); PENNA, Christopher, Guilford, CT 06437 (US)
(74) Representative: Soames, Candida Jane

(56) References cited:
- EP-A1- 0 648 476
- EP-A1- 0 760 230
- EP-A1- 1 563 791
- EP-A2- 2 236 098
- AU-B2- 2002 300 129
- US-A- 6 032 849

## Description

### CROSS-REFERENCE TO RELATED APPLICATIONS

This application claims the benefit of and priority to U.S. Provisional Patent Application No. 61/779,873, filed March 13, 2013.

### BACKGROUND

### Technical Field

The present disclosure relates to surgical stapling apparatuses. More particularly, the present disclosure relates to surgical stapling apparatuses including knife drive lockout mechanisms.

### Description of Related Art

Surgical stapling apparatuses that are configured to staple and, subsequently, sever tissue are well known in the art. Such stapling apparatuses, typically, include, a housing and an elongated member that extends from the housing. In certain instances, a multi use loading unit (MULU) that includes a reload may be configured to releasably couple to a distal end of the elongated member. Alternatively, the reload may be fixedly supported at the distal end of the elongated member. In either of the aforementioned reload configurations, an anvil and cartridge may be provided on jaws of the reload and configured to staple tissue. A knife (or other suitable device) may be utilized to sever the stapled tissue. The knife may be actuated via one or more actuation devices operably associated with the surgical stapling apparatus and translated through the anvil and cartridge to sever the staple tissue. EP 1 563 791 A1 discloses a disposable loading unit comprising a locking mechanism configured to prevent distal movement of the drive member whilst the loading unit is not attached to a stapling apparatus.

While the aforementioned reload configurations provide numerous advantages, it may be desirable to prevent inadvertent firing of the knife of the surgical stapler when a staple cartridge is spent.

### SUMMARY

As can be appreciated, surgical stapling apparatuses that include knife drive lockout mechanisms may prove useful in the surgical arena.

Embodiments of the present disclosure are described in detail with reference to the drawing figures wherein like reference numerals identify similar or identical elements. As used herein, the term "distal" refers to the portion that is being described which is further from a user, while the term "proximal" refers to the portion that is being described which is closer to a user.

The present invention is defined in independent claim 1 and certain optional features thereof are defined in the dependent claims. In so far as the terms "invention", "example", "aspect", and "embodiment" are used herein, this will be interpreted in such a way that the only protection sought is for the invention as claimed..

### BRIEF DESCRIPTION OF THE DRAWING

Various embodiments of the present disclosure are described hereinbelow with references to the drawings, wherein:
Fig. 1 is a perspective view of an example powered surgical stapling apparatus;
Fig. 2 is a perspective view of an example manual surgical stapling apparatus;
Fig. 3A is a perspective view of an example reload of Figs. 1 and 2 including an example drive lockout mechanism;
Fig. 3B is an exploded, perspective view of the reload of Fig. 3A with the parts separated;
Fig. 4A is a partial, perspective view of an example removable cartridge including a spring clip shown in an extended configuration;
Fig. 4B is a perspective view of a proximal portion of the cartridge with the spring clip of Fig. 4A removed;
Fig. 5 is a perspective view of the spring clip of Fig. 4A;
Fig. 6 is an example perspective view of an anvil uncoupled to a corresponding jaw member to illustrate a recess configured to receive the spring clip therein;
Fig. 7 is a perspective view of an example pivot beam that is configured to releasably engage the spring clip;
Fig. 8 is a partial, perspective view of the example anvil and cartridge with a top portion of the anvil being removed to illustrate a knife in a pre-fired configuration and the spring clip and pivot beam in an engaged configuration;
Fig. 9 is a cut-away view taken along line-segment 9-9 in Fig. 8;
Figs. 10-12 are perspective views illustrating an example firing sequence of the knife through the cartridge and anvil;
Fig. 13 is a perspective view of an example reload including a drive lockout mechanism;
Fig. 14 is a perspective view of the reload and a cartridge depicted in Fig. 13 uncoupled from one another;
Fig. 15 is an exploded, perspective view of an example reload with the parts separated and removed;
Fig. 16 is an exploded, perspective view of an example cartridge assembly with parts separated;
Fig. 17 is an exploded, bottom view of a sled of an example cartridge with parts separated;
Fig. 18 is a bottom view of the sled of Fig. 17 in an assembled configuration;
Fig. 19 is an exploded, rear perspective view of an example sled with parts separated;
Fig. 20 is a rear perspective view of the sled of Fig. 19 in an assembled configuration;
Fig. 21 is an enlarged area of detail of Fig. 15 illustrating a latch;
Fig. 22 is a perspective view of the latch depicted in Fig. 21 shown inverted;
Fig. 23 is perspective view of an example reload with parts removed;
Fig. 24 is an enlarged area of detail of Fig. 23;
Fig. 25 is a partial, perspective view of an example reload with parts removed illustrating a pivot assembly;
Fig. 26 is a cross-sectional view taken along line portion 26 in Fig. 25;
Figs. 27 is a partial, cross-sectional view of an example cartridge illustrating the cartridge being installed to a corresponding jaw member;
Fig. 28 is a partial, cross-sectional view of an example cartridge illustrating the cartridge fully installed to the corresponding jaw member;
Fig. 29 is a partial, cross-sectional view of an example cartridge illustrating the cartridge being approximated towards;
Fig. 30 is a partial, cross-sectional view of an example cartridge illustrating the anvil and cartridge being in a fully approximated configuration;
Fig. 31 is a partial, cross-sectional view of an example cartridge illustrating a firing motion of a knife of the reload;
Fig. 32 is a partial, cross-sectional view of an example cartridge illustrating the knife being retracted back to a pre-fired configuration;
Fig. 33 is a partial, cross-sectional view of an example cartridge illustrating the anvil and cartridge in an open configuration and the knife in the retracted configuration;
Fig. 34 is a partial, cross-sectional view of an example cartridge illustrating the knife in the retracted configuration and the latch in position for removal of the reload from a trocar;
Fig. 35 is a partial, cross-sectional view of an example cartridge illustrating the knife in the retracted configuration and the latch in a locked out configuration;
Fig. 36 is perspective view of an example reload with parts removed and including a drive lockout mechanism according to another embodiment of the present disclosure;
Fig. 37 is an enlarged area of detail of Fig. 36;
Fig. 38 is an exploded, perspective view of the reload depicted in Fig. 36;
Fig. 39 is an exploded, perspective view of an example cartridge assembly with parts separated;
Fig. 40 is an enlarged area of detail of Fig. 38 illustrating a latch;
Fig. 41 is a perspective view of the latch depicted in Fig. 21 shown inverted;
Fig. 42 is a perspective view of an example reload with parts removed illustrating a pivot assembly;
Fig. 43 is a perspective view of an example reload with parts removed including a portion of the pivot assembly to illustrate a distal end of a knife assembly;
Fig. 44 is a partial, cross-sectional view of an example reload with the cartridge not installed on a corresponding jaw member;
Fig. 45 is a partial, cross-sectional view of an example cartridge installed on a corresponding jaw member;
Fig. 46 is a partial, cross-sectional view of an example cartridge illustrating a knife being translated therethrough;
Fig. 47 is a partial, cross-sectional view of an example cartridge illustrating a knife in a retracted configuration and locked out;
Fig. 48 is a perspective view of a reload with parts removed and including a drive lockout mechanism according to the invention;
Fig. 49 is an enlarged area of detail of Fig. 48;
Fig. 50 is a partial, cut-away view of a cartridge of the invention shown in a pre-fired configuration;
Fig. 51 is an exploded, perspective view of the cartridge of the invention with parts removed;
Fig. 52 is a perspective view of an actuator of the cartridge depicted in Figs. 50 and 51;
Fig. 53 is a perspective view of the actuator depicted in Fig. 52 shown inverted;
Fig. 54 is a perspective view of a rotating interlock of the cartridge of the invention;
Fig. 55 is a top elevational view of the cartridge of the invention;
Fig. 56 is a cut-away view taken along line section 56-56 shown in Fig. 55;
Fig. 57 is a partial, perspective view of the cartridge of the invention with parts removed illustrating a knife during a firing sequence;
Fig. 58 is a cut-away view taken along line section 58-58 shown in Fig. 57;
Fig. 59 is a partial, perspective view of the cartridge of the invention with parts removed illustrating the knife in a locked-out configuration;

### DETAILED DESCRIPTION

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In accordance with the instant disclosure, various drive lockout mechanisms are disclosed herein and are configured for use with reloads that are adapted to couple to one or more types of surgical stapling apparatuses. The various drive lockout mechanisms are configured to prevent misfiring of a knife without a cartridge installed, or firing with a spent cartridge installed.

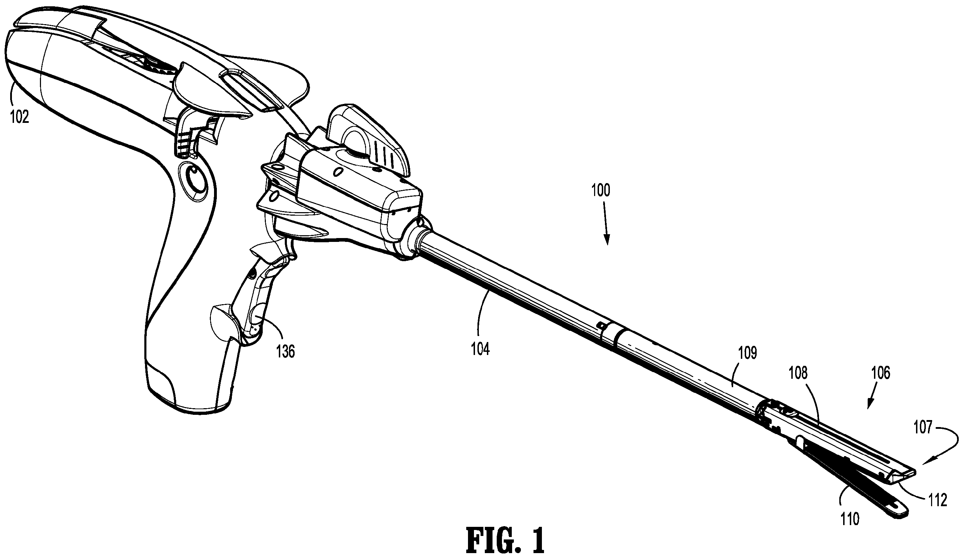

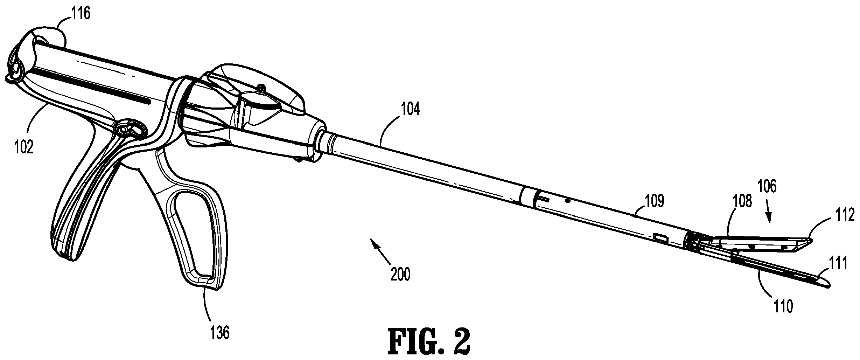

Fig. 1 illustrates a powered surgical stapling apparatus shown generally as 100. Fig. 2 illustrates a manual surgical stapling apparatus shown generally as 200. The powered apparatus includes one or more motors and an internal or external power source, whereas the manual apparatus has a movable handle 136 and a mechanism for driving the functions of the apparatus. See U.S. Patent Nos. 5,865,361; 5,782,396; International WO 04/032,760; U.S. Patent Publication No. 2010/0276741; and U.S. Patent Application Ser. No. 13/444,228.

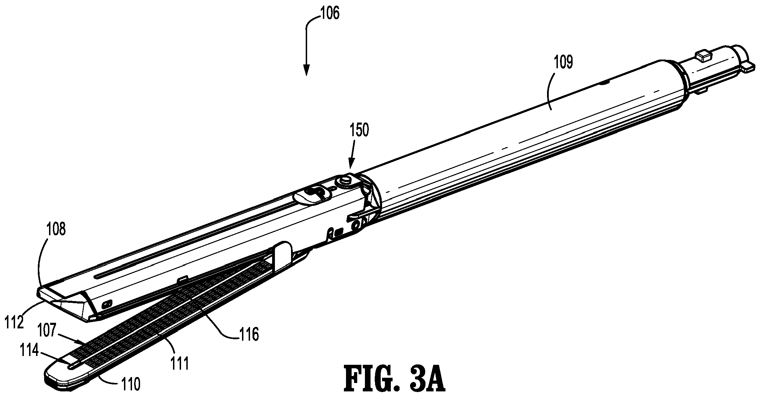

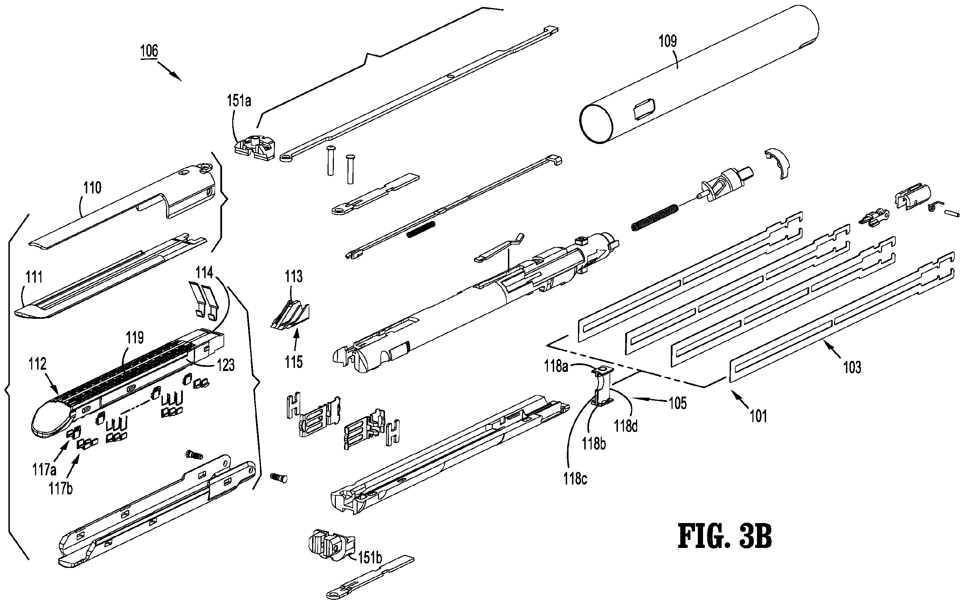

Briefly, the surgical stapling apparatus 100, 200 includes a housing 102 a retractor 116, a firing mechanism 116 (Fig. 2), an elongated member 104 extending from housing 102, and a reload 106 that is releasably coupled to a distal end of elongated member 104. Reload 106 includes a proximal shaft portion 109 having a distal end which a tool assembly including first and second jaw members 108, 110. First jaw member 108 is configured to support a cartridge 112 which includes a plurality of fasteners 117a and a corresponding plurality of pusher members 117b that are engaged with fasteners 117a (see Fig. 3B). Cartridge 112 includes one or more retention slots 119 that extend longitudinally along a tissue contacting surface 121 of a cartridge housing 123 and are configured to house fasteners 117a (Fig. 3B). Cartridge housing 123 (Fig. 3B) is configured to releasably couple to first jaw member 108 via one or more suitable coupling methods. A removable and replaceable cartridge assembly is disclosed in U.S. Patent Application Ser. No. 13/280,880 entitled Multi-Use Loading Unit. In any of the embodiments disclosed herein, a removable and replaceable cartridge assembly may be coupled to a jaw using detents, latches, clips and the like. Second jaw member 110 is provided with an anvil 111 (as best seen in Fig. 3B) which defines a plurality of buckets or depressions 107 (see Fig. 3A) that are configured to receive corresponding fasteners 117a when fasteners 117a are ejected from cartridge 112.

Fig. 3B illustrates components that are housed within shaft 109. A drive member includes a drive beam 103 having a working end 101 which supports a knife 105. Working end 101 includes an I-beam configuration having top and bottom flanges 118a, 118b and includes a distal abutment surface 118c which engages a central support wedge 113 of actuation sled 115 (see Fig. 3B). Working end 101 is configured to move through a knife channel 114 (Fig. 3B) defined in cartridge 112 from a retracted position to an advanced position for severing stapled tissue positioned between the jaw 108, 110. Knife blade 105 travels slightly behind actuation sled 115 during a stapling procedure such that an incision is formed in tissue after the tissue has been stapled.

A pivot assembly 150 (Fig. 3A) is provided at a distal end of shaft 109 and couples first and second jaw members 108, 110 to shaft 109. Pivot assembly 150 includes lower and top portions 151b, 151a that are operably coupled to one another and the tool assembly to facilitate articulation of the tool assembly about an axis transverse to a longitudinal axis of shaft 104 (Fig. 3B).

For a more detailed discussion of the construction and operation of reload 106, reference may be made to U.S. Patent Nos. 5,865,361 and 7,225,963.

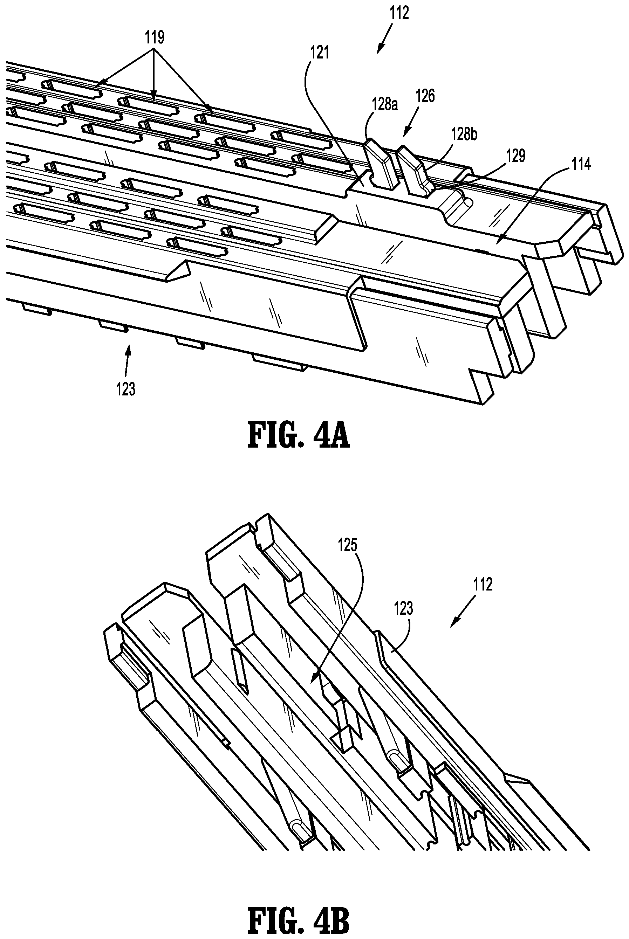

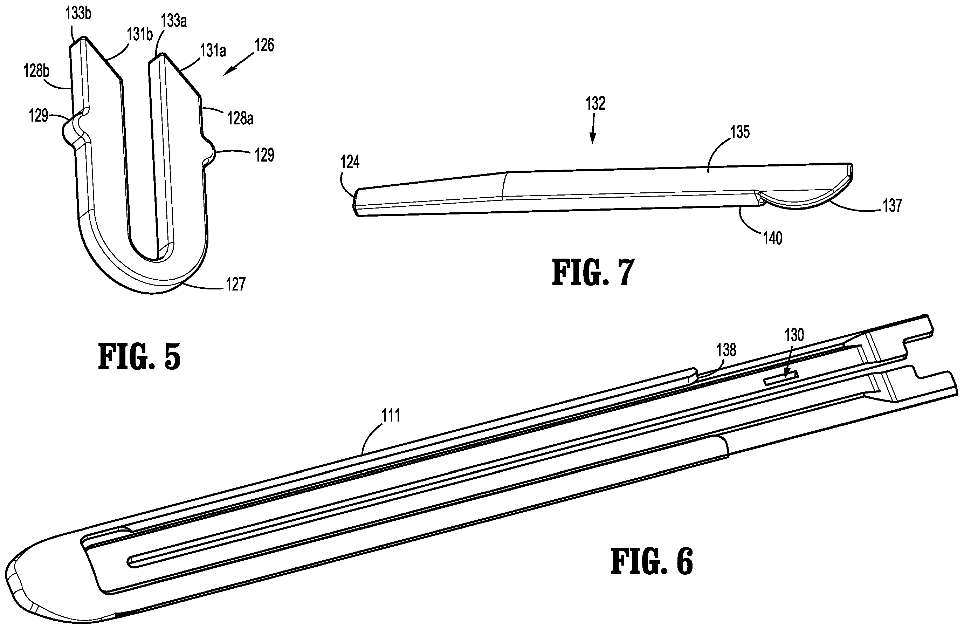

In accordance with the instant disclosure, reload 106 includes a locking mechanism according to an embodiment of the instant disclosure. Specifically, and with reference to Figs. 4A-4B, cartridge housing 123 includes one or more recesses 125 (Fig. 4B) of suitable configuration that are configured to receive and/or operably house one or more resilient members 126 (see Fig. 5). A single recess 125 which opens onto a top surface of the cartridge 112 is shown in the illustrated embodiment. Recess 125 is configured to allow flexure of legs 128a, 128b of the resilient member 126 within the confines of recess 125.

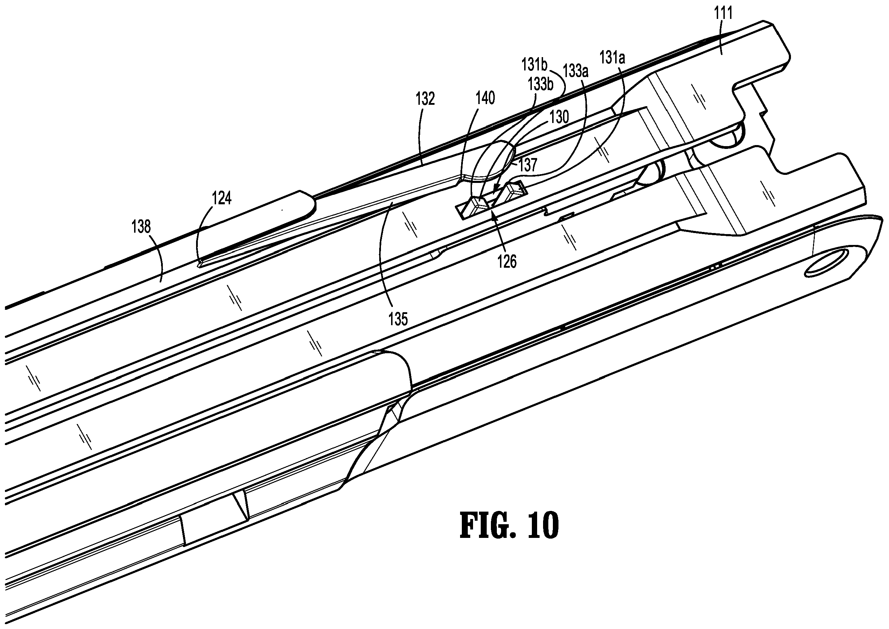

Continuing with reference to Fig. 5, resilient member(s) 126 may be formed from any suitable resilient material including but not limited to plastic, rubber, metal, etc. In the illustrated embodiment, resilient member is made from a relatively soft plastic and formed into a spring-clip 127. Spring-clip 127 is movable from an extended position (Fig 4a) to a retracted position (Fig. 10) and includes a generally arcuate configuration and is defined by opposing legs 128a, 128b that form a generally "U" configuration; this "U" configuration facilitates positioning spring-clip 127 within recess 125. In accordance with the instant disclosure, prior to use of cartridge 112, spring-clip 127 extends a predetermined distance above tissue contacting surface 121. To this end, one or both of legs 128a, 128b may include one or more flanges 129 (Figs. 4A and 5) that are configured to releasably engage a surface 121 of cartridge 112 adjacent proximal end of the tissue contacting surface of cartridge 112 (Fig. 4A). In the illustrated embodiment, each of legs 128a, 128b includes a single flange 129. Moreover, one or both of legs 128a, 128b may have beveled or angled ends 131a, 131b positioned for engagement with top flange 118a of the knife 105 when knife 105 is advanced from a retracted position towards an advanced position. In the illustrated embodiment, each of sidewalls 128a, 128b includes angled surfaces 131a, 131b that culminate at tips 133a, 133b. Resilient member(s) 126 are configured for insertion through a corresponding recess 130 disposed on anvil 111 (Figs. 6 and 10). Recess 130 on anvil 111 is in vertical registration with recess 125 of cartridge 112 to facilitate insertion of resilient member 126 within recess 130.

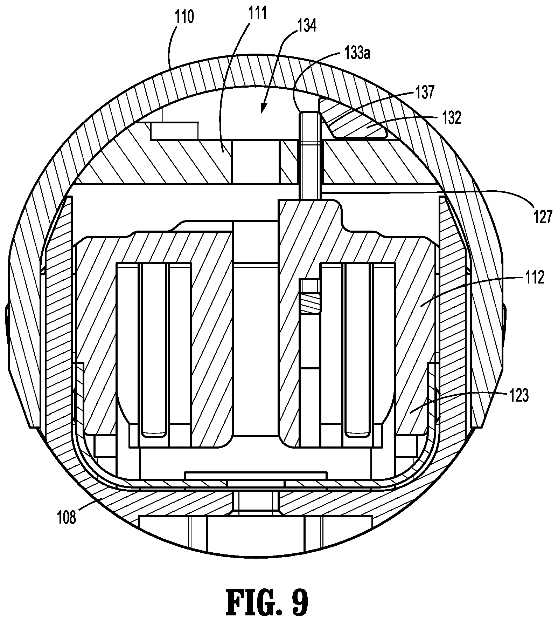

Referring now to Fig. 6, anvil 111 is illustrated uncoupled from jaw member 110 to illustrate recess 130. Recess 130 is of suitable configuration to receive spring-clip 126 therein. Specifically, angled end surfaces 131a, 131b are configured for positioning within recess 130 when a newly inserted (e.g., a pre-fired) cartridge 112 is coupled to jaw member 108 and jaw members 108, 110 are approximated, see Fig. 9 for example. In the extended configuration, spring clip 127 prevents movement of the locking member 132 (Fig. 7) within an internal cavity 134 (see Fig. 9 for example) of jaw member 110 to a blocking position as will be described in further detail below.

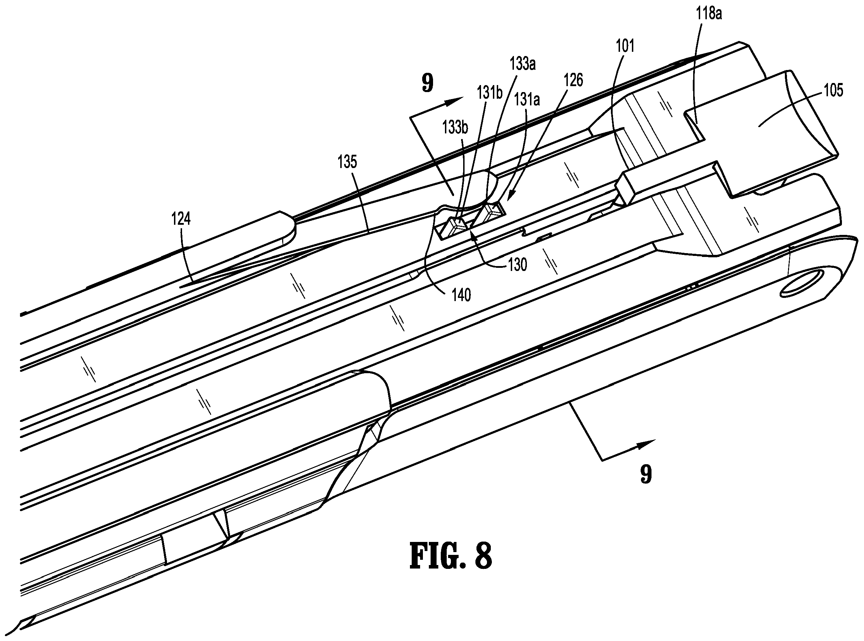

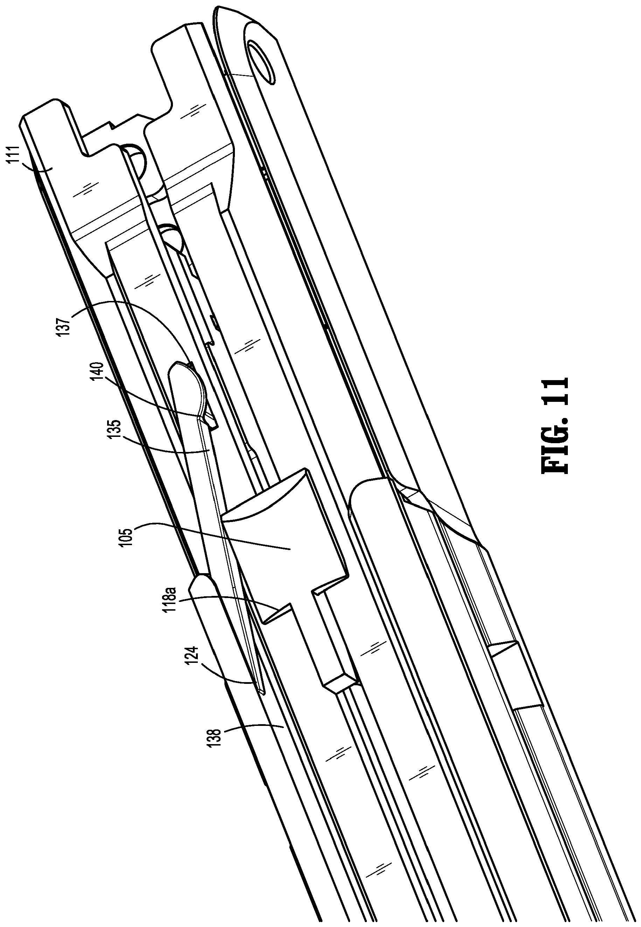

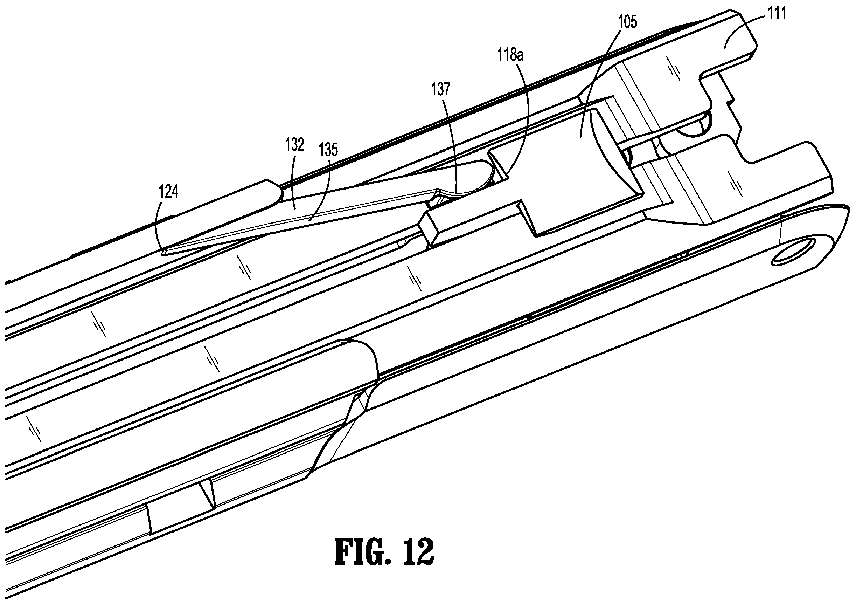

With reference to Fig. 7, locking member 132 has a generally elongated configuration and includes a distal end 124 that is operably coupled to a side wall 138 of anvil 111 to facilitate movement of the locking member 132 upwardly and transversely from an outer surface of anvil 111 towards the center of anvil 111 to a position to obstruct movement of the working end 101 of drive member (Fig. 8). A cam surface 137 is disposed at a proximal end 140 of locking member 132 and is configured to engage top flange 118a disposed on a top portion 144 of knife 105 (Figs. 8 and 11-12). Engagement between cam surface 137 and top flange 118a prevents knife 105 from moving distally past locking member 132 after the cartridge 112 has been fired as will be described in further detail below. A sidewall 135 of locking member 132 is configured to contact a top portion 144 of knife 105 as knife 105 is moved from the advanced configuration to the retracted configuration (see Fig. 11 for example) to move the locking member 132 from the locking position (Fig. 12) to a non-blocking position to allow the knife to move from the retracted position.

In use, when cartridge 112 is not coupled to jaw member 108, locking member 132 is in the blocking position for engaging knife 105 (or component associated therewith, e.g., top flange 118a). That is, cam surface 13 7 is flush with the plane of translation of knife 105 such that an end of locking member 132 engages top flange 118a to prevent knife 105 from traveling distally past locking member 132 (see Fig. 12). Thus, the locking member 132 prevents firing of apparatus 100, 200 when a cartridge 112 has not been inserted into jaw 108.

When cartridge 112 is coupled to jaw member 108, locking member 132 pivots upwardly as a result of contact with resilient member 126 (see Figs. 8-9). This contact allows working end 101 of the drive member to travel distally past the locking member 132 when knife 105 is fired (Fig. 10). Specifically, this contact raises the cam surface 137 off the plane of translation of working end 101 and above top flange 118a, which, in turn, prevents engagement therebetween so as to allow knife 105 to travel distally past locking member 132 when working end 101 is advanced. Essentially, top flange 118a slides beneath cam surface(s) 137 as knife 105 is translated distally.

Contact between top flange 118a and tips 133a, 133b and/or angled surfaces 131 a, 131b as the working end 101 is advanced causes flanges 129 to disengage from tissue contacting surface 121 of cartridge 112, which, in turn, causes tips 133a, 133b and/or angled surfaces 131a, 131b to fall beneath the translation plane of working end 101 (Fig. 10). This allows locking member 132 to return to its initial configuration (Fig. 11) obstructing distal movement of the working end 101.

As working end 101 is moved proximally back to its retracted configuration, top portion 144 contacts a sidewall 135 (Fig. 8) of locking member 132 to pivot locking member 132 sideways towards sidewall 138 of anvil 111. Once top portion 144 has been moved proximally past cam surface(s) 137 of locking member 132, locking member 132 again returns to its initial configuration. In its initial configuration, cam surface 137 is flush with the plane of translation of working end 101 and positioned to engage top portion 144 of working end 101 (Fig. 12).

The unique configuration of locking member 132 and resilient member 126 overcomes the aforementioned drawbacks that are, typically, associated with conventional surgical stapling apparatus. Specifically, the locking member 132 prevents firing of the stapling apparatus 100, 200 when a cartridge 112 is not coupled to jaw 108 or when cartridge 112 has already been fired.

From the foregoing and with reference to the various figure drawings, those skilled in the art will appreciate that certain modifications can also be made to the present disclosure without departing from the scope of the same. For example, while the surgical stapling apparatuses 100, 200 have been described herein as including one locking member 132 and one corresponding resilient member 126, it is within the purview of the present disclosure to utilize two or more locking members 132 and corresponding resilient members 126.

Additionally, while surgical stapling apparatuses 100, 200 have been described herein utilizing a reload 106 the drive lockout mechanism described above can be supported on the tool assembly of any stapler having a replaceable cartridge.

In addition, reloads that include other types of locking mechanisms may also be utilized with surgical stapling apparatuses 100, 200. The following reloads are similar in concept and design to reload 106. Accordingly, only those features unique to the hereinafter described embodiments of reloads are described in detail.

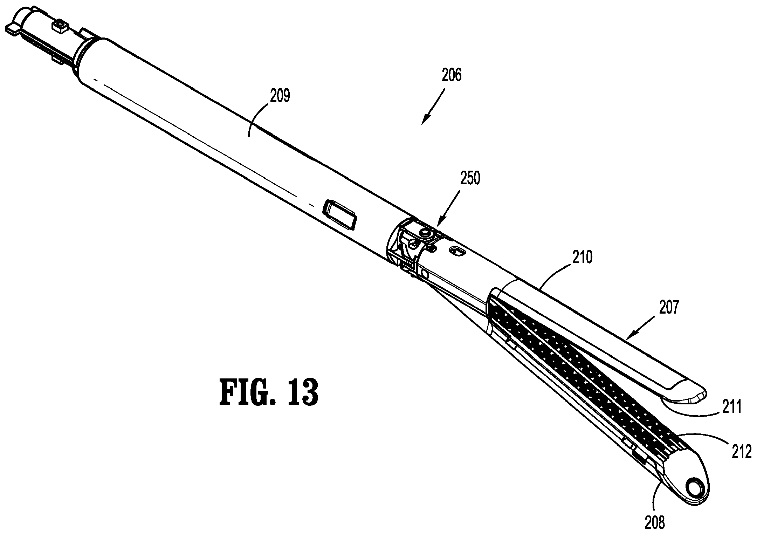

With reference to Figs. 13-35, and initially with reference to Figs. 13-14, an example reload 206 includes a locking mechanism according to another embodiment of the instant disclosure and is configured for use with surgical stapling apparatuses 100, 200 (Figs. 1 and 2).

Reload 206 is generally as described above but the configuration of the locking mechanism has changed as described below. Reload 206 includes shaft 209 that supports a tool assembly 207 including jaw members 208, 210, respectively. Jaw member 208 is configured to releasably engage a cartridge 212 and jaw member 210 is provided with an anvil 211. Jaw members 208, 210 function in a manner as described above with respect to jaw members 108, 110.

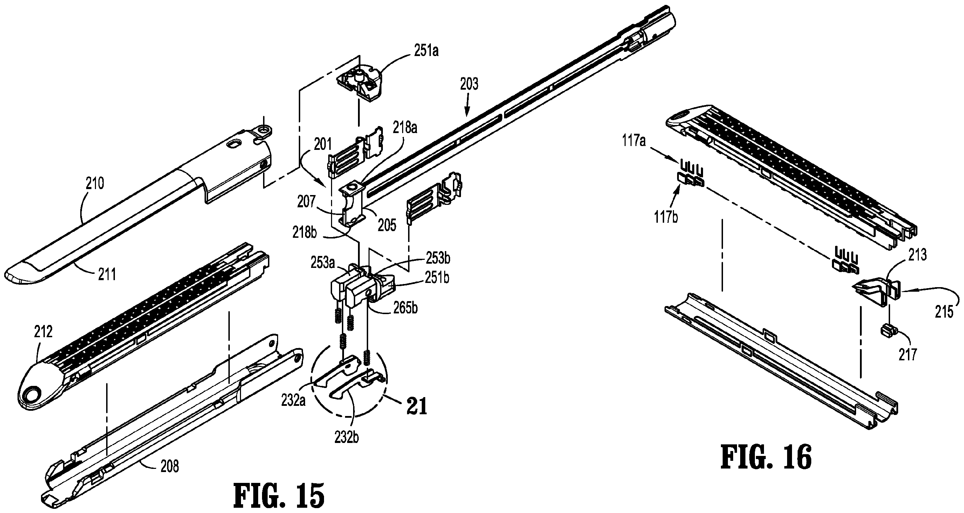

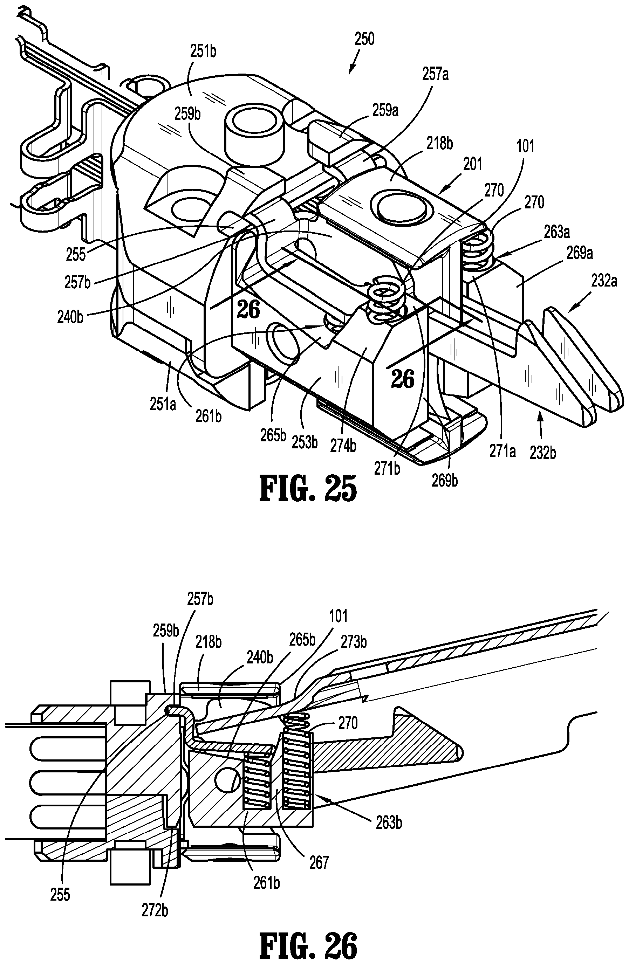

A pivot assembly 250 is configured to function in a manner as described above with respect to pivot assembly 150 and includes top and lower portions 251a, 251b (see Fig. 15 for example). Unlike lower portion 151b, however, lower portion 251b is configured to operably support a pair of latches 232a, 232b that are operable to lock a working end 101 of a drive member in a retracted configuration. Specifically, lower portion 251b includes a pair of distally extending leg members 253a, 253b (Figs. 15 and 25). Leg members 253a, 253b are spaced-apart a predetermined distance from one another to receive knife 205 (Fig. 15) so as to allow working end 101 to move through a firing sequence of surgical stapling apparatuses 100, 200, as will be described in greater detail below.

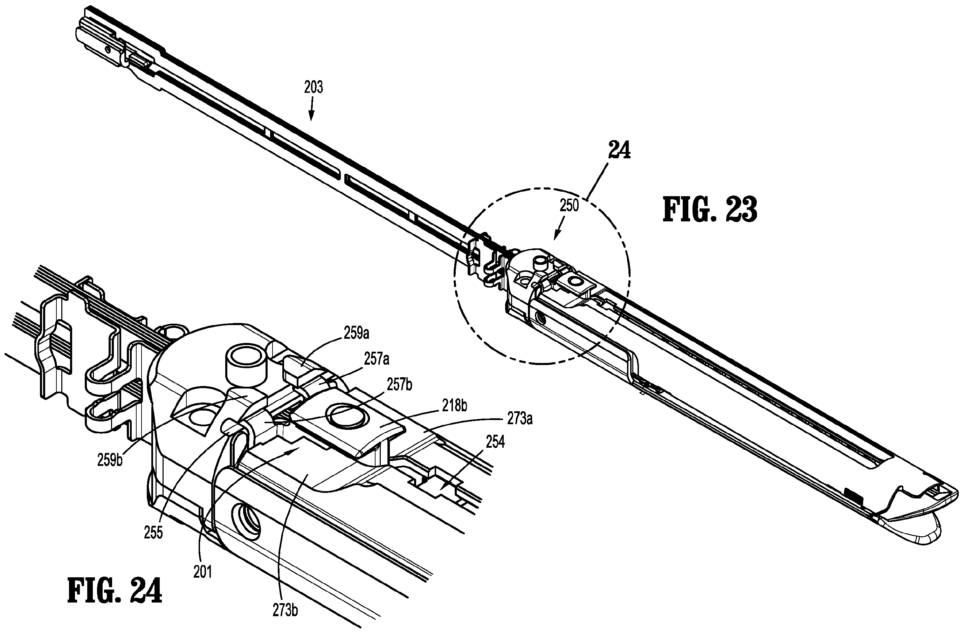

A shelf 255 (Figs. 24-25) of suitable configuration is provided on lower portion 251b and is positioned proximally with respect to latches 232a, 232b. Shelf 255 extends across lower portion 251b and is configured to support finger portions 257a, 257b of latches 232a, 232b, respectively. A pair of spaced-apart holders 259a, 259b are provided on lower portion 251b and are positioned adjacent shelf 255. Holders 259a, 259b extend distally from lower portion 251b such that a distal face of holders 259a, 259b aligns with a distal edge of shelf 255 (Fig. 26). Holders 259a, 259b are configured to engage finger portions 257a, 257b to maintain direct contact between finger portions 257a, 257b and shelf 255. In embodiments, holders 259a, 259b may be replaced with a single holder that extends along an entire length of the shelf 255.

Continuing with reference to Fig, 25, leg member 253b includes a generally flat medial portion 265b (Figs. 15 and 25) that defines a cavity 261b of suitable configuration defined therein that is configured to house a spring 267, e.g., a compression spring, (Fig. 26). Medial portion 265b is angled in a direction towards a toe portion 269b of leg member 253b. Toe portion 269b extends distally from medial portion 265b and includes a generally flat top surface 271b (Fig. 25) that is elevated a predetermined distance above medial portion 265b. Top portion 271b is configured to contact an offset flange portion 273b (Fig. 24) of cartridge 212. A proximal face 272b (Fig. 26) of toe portion 269b is angled toward medial portion 265b and a sidewall 274b is angled in an outward direction (Fig. 25) away from top surface 271b. A cavity 263b of suitable configuration is defined in toe portion 269b and is configured to house an optional spring 270, e.g., a compression spring, (Fig. 26). Spring 270 may be configured to bias cartridge 212 to the open position.

A second toe portion 269a extends distally from a medial portion (not explicitly shown) of leg member 253a and defines a cavity 263a that is configured to house spring 270 (Fig. 25). Toe portion 269a includes a generally flat top surface 271a (Fig. 25) that contacts a corresponding offset flange portion 273a (Fig. 24) of cartridge 212. The medial portion of leg member 253a includes a cavity (not explicitly shown) that is configured to house a spring 270.

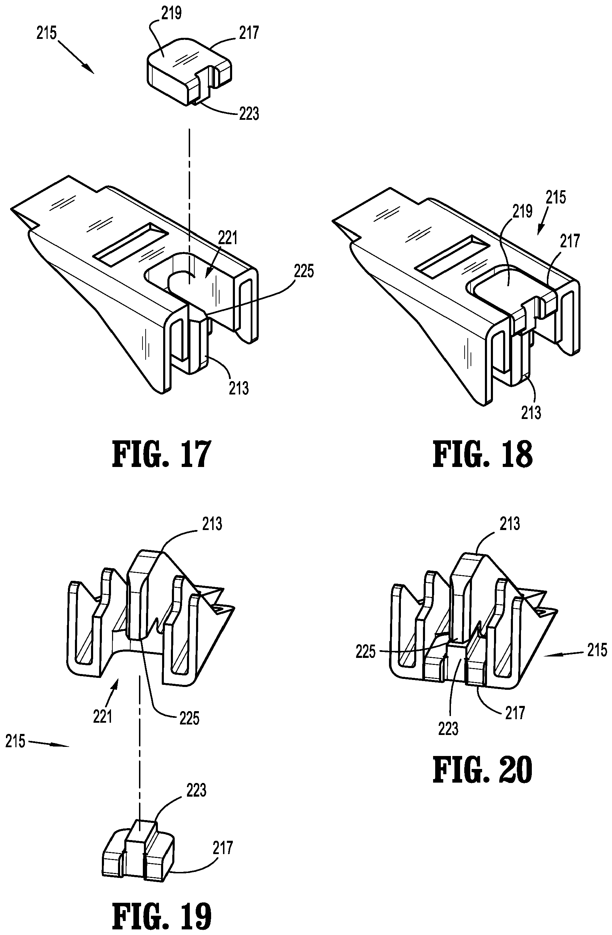

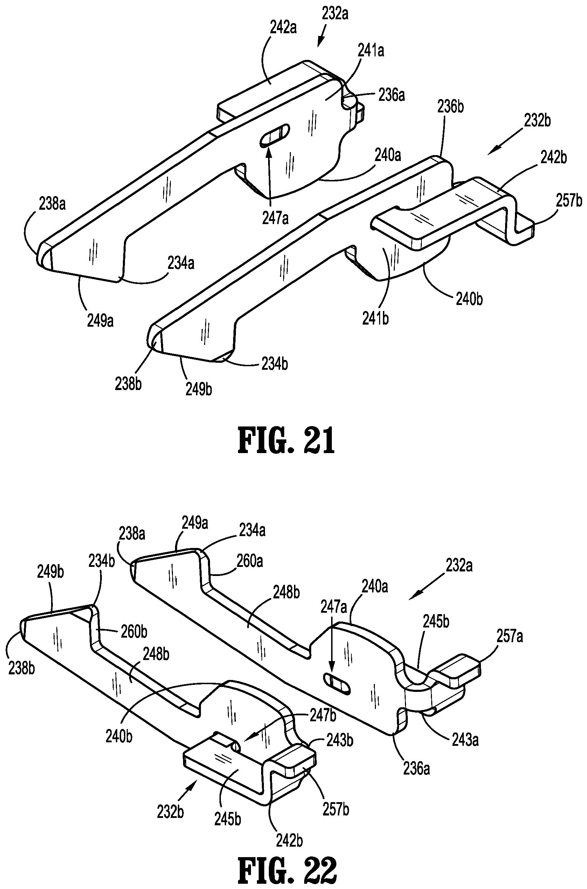

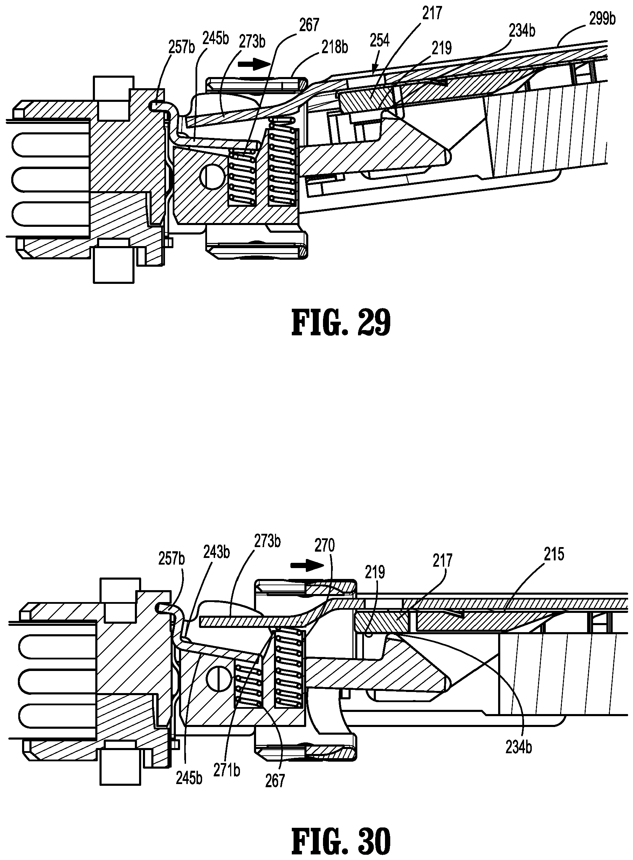

Referring to Figs. 16-19, actuation sled 215 is similar to actuation sled 115 and includes a central support 213. Unlike actuation sled 115, however, actuation sled 215 includes a blocking member 217. Blocking member 217 may be monolithically formed with actuation sled 215 or blocking member 217 may be a separate component that is coupled to actuation sled 215 via one or more suitable coupling methods, e.g., press-fit, friction-fit, adhesive, etc. In the illustrated embodiment, actuation sled 215 and blocking member 217 are formed as separate components via an injection molding process and, subsequently, coupled to one another via a press-fit. Blocking member 217 includes a generally curvilinear base portion 219 that complements a corresponding recess 221 provided on a bottom portion of actuation sled 215. A detent 223 is provided on base portion 219 of actuation sled 215 and abuts a bottom surface 225 of central wedge 213 when actuation sled 215 is in an assembled configuration (Figs. 18 and 20). Blocking member 217 is configured to contact a pair of distal protuberances 234a, 234b (Figs. 21-22) of latches 232a, 232b when a loaded cartridge 212 is coupled to jaw member 208 (Fig. 30). In further embodiments, one or more latches may be used.

Referring now to Figs. 21-22, latches 232a, 232b may be formed via any suitable process and include proximal ends 236a, 236b and distal ends 238a, 238b, respectively. Body portions 240a, 240b are provided at respective proximal ends 236a, 236b and are configured to contact flange 218b when knife 205 is in a retracted configuration (Figs. 26-29 and 33-34). This contact between flange 218b and body portions 240a, 240b maintains latches 232a, 232b in an unlatched configuration.

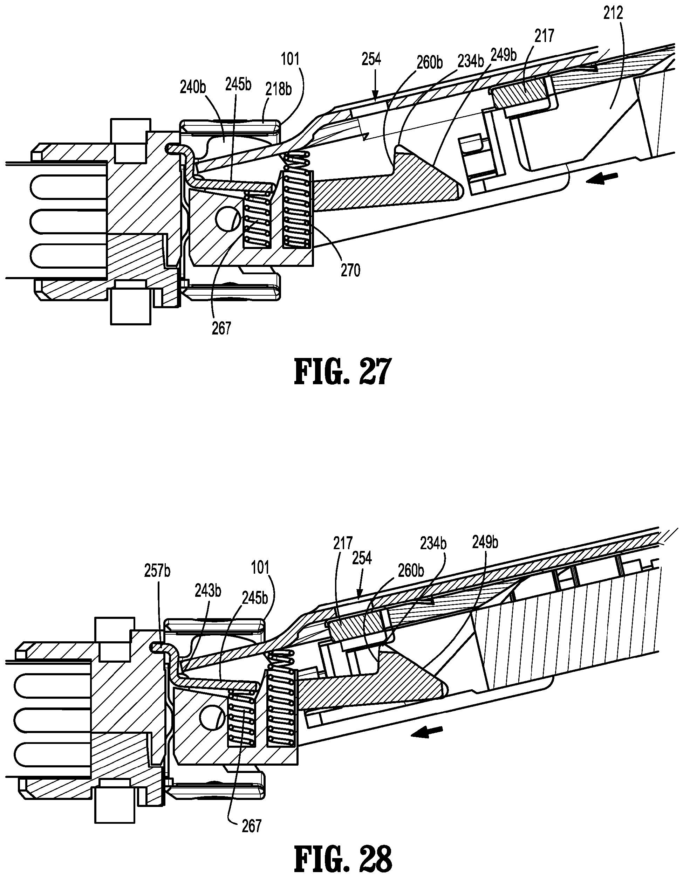

Lateral extensions 242a, 242b of latches 232a, 232b include generally arcuate shoulder portions 243a, 243b that extend from proximal ends 236a, 236b and have respective arms 245a, 245b that abut sidewalls 241a, 241b of body portions 240a, 240b. Distal ends of arms 245a, 245b are received within corresponding apertures 247a, 247b (Figs. 21-22) defined in lateral extensions 242a, 242b. Finger portions 257a, 257b extend in a generally orthogonal direction from shoulder portions 243a, 243b and proximally toward shelf 255 for engagement with corresponding holders 259a, 259b. Arms 245a, 245b are configured to engage springs 267 provided on leg members 253a, 253b to bias latches 232a, 232b in a downwardly direction (Fig. 28).

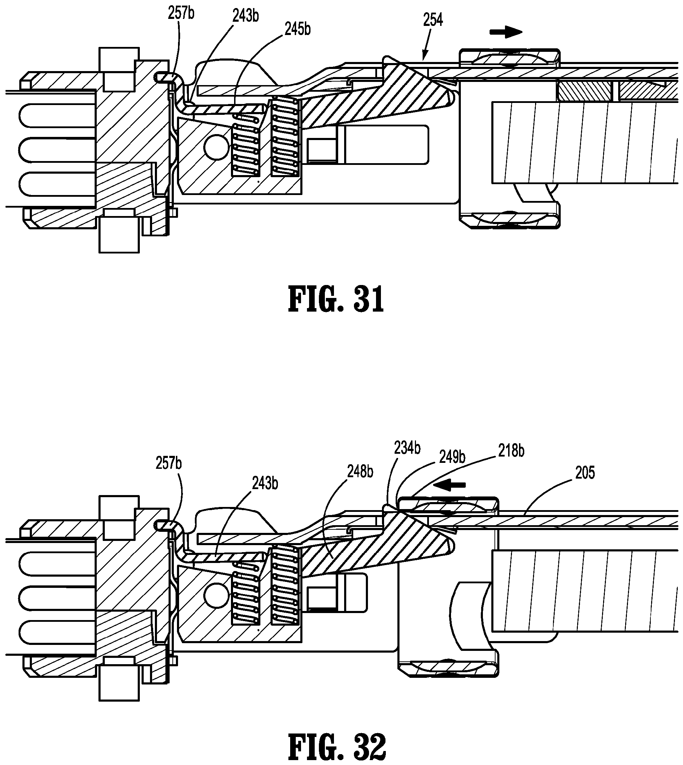

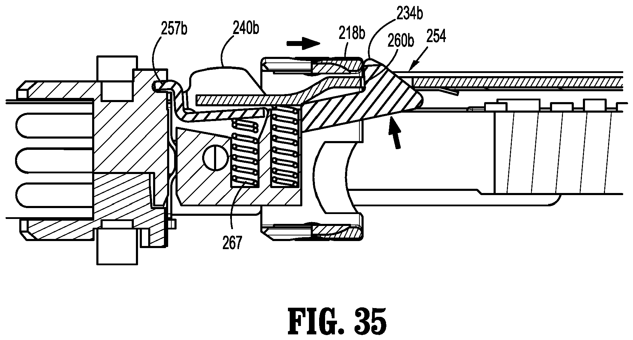

Continuing with reference to Figs. 21-22, extending distally from body portions 240a, 240b are elongated members 248a, 248b from which trailing surfaces 260a, 260b extend in a generally orthogonal direction and culminate at protuberances 234a, 234b. Protuberances 234a, 234b are configured to selectively engage a recess 254 that is provided on an underside of jaw member 208, see Fig. 24 in combination with Fig. 31. Protuberances 234a, 234b and/or trailing surfaces 260a, 260b are configured to engage flange 218b of working end 201 of the drive member when protuberances 234a, 234b are engaged with recess 254(Fig. 35). Extending distally from protuberances 234a, 234b are angled leading surfaces 249a, 249b that are configured to contact flange 218b of knife 205 when knife 205 is moved proximally back to the retracted configuration. Leading surfaces 249a, 249b allow flange 218b to slide past protuberances 234a, 234b to allow the working end 201 to be move proximally back to the retracted configuration(Fig. 32).

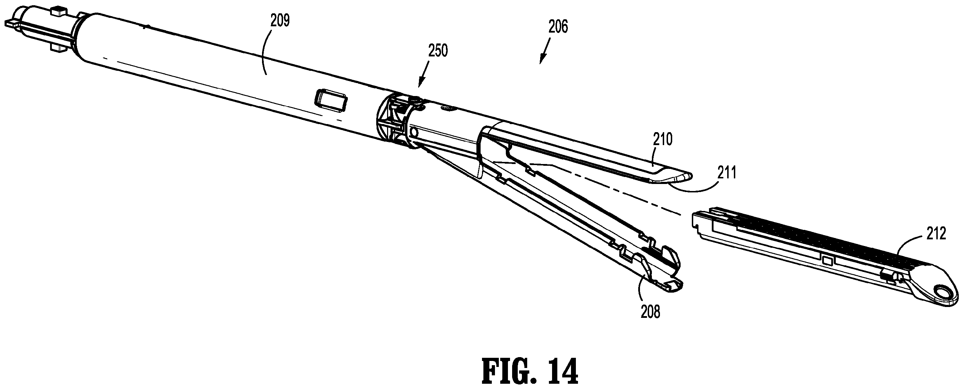

Operation of surgical stapling apparatuses 100, 200 that utilize reload 206 is described herein. Initially, jaw members 208, 210 may be in an open configuration to load cartridge 212 onto jaw member 208 (Figs. 14 and 26-27). In the open configuration, working end 201 is in a fully retracted configuration and flange 218b contacts body portions 240a, 240b. Moreover, arm portion 245b is pressed against springs 267.

Thereafter, cartridge 212 may be inserted in jaw member 208. In the loaded configuration, blocking member 217 is positioned over recess 254 and in contact with protuberances 234a, 234b so as not to allow protuberances 234a, 234b to engage recess 254 prior to actuation sled 215 and/or the drive member being fired (Figs. 28-29).

Subsequently, reload 206 including jaw members 208, 210 may be inserted through a portal, e.g., a trocar (or other suitable device), and positioned within a cavity of a patient adjacent tissue of interest. Tissue may be positioned between jaw members 208, 210 and jaw members 208, 210 may be approximated towards one another to grasp tissue for stapling thereof.

When the working end 201 is advanced to staple and sever tissue, flange 218b translates distally and moves out of engagement with body portions 240a, 240b. However, because blocking member 217 covers recess 254 and contacts with protuberance 234b, the working end 210 is free to continue to move distally and contact central cam wedge 213 of actuation sled 215, which, in turn, moves blocking member 217 out of contact with protuberances 234a, 234b. Accordingly, protuberances 234a, 234b are allowed to engage recess 254 (Fig. 31) as a result of bias of spring 267.

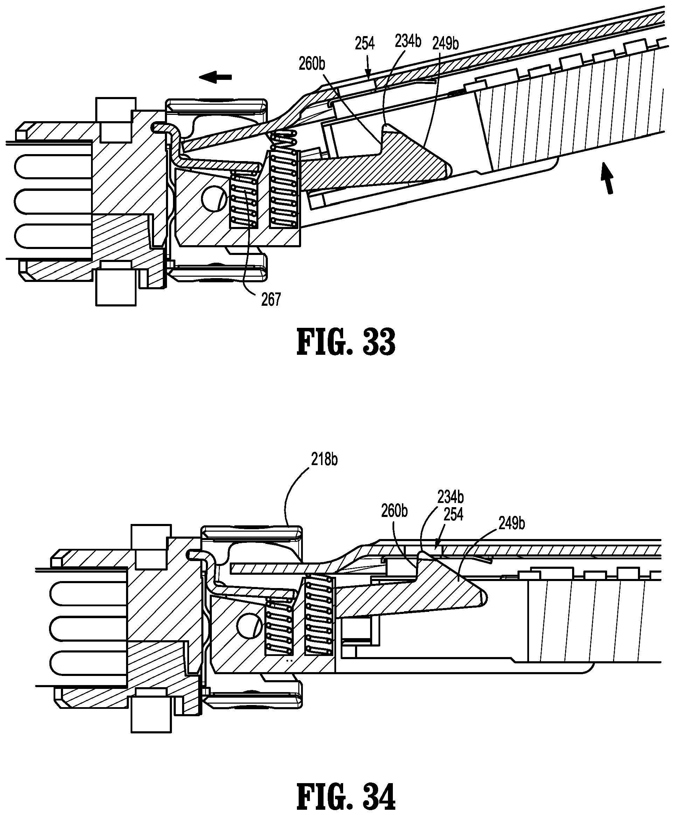

Subsequent to stapling and severing tissue, the working end 210 may be moved proximally and returned to its fully retracted configuration. Specifically, flange 218b of knife 205 contacts and slides past leading surfaces 249a, 249b so as to allow the working end 210 to be moved back to its fully retracted continuation (Figs. 32-33). Flange 218b of knife 205 contacts body portions 240a, 240 and protuberances 234a, 234b are prevented from engaging recess 254. Accordingly, jaw members 208, 210 may be approximated towards one another for removal through the portal without interference from protuberances 234a, 234b (Fig. 34). That is, because the protuberances 234a, 234b are prevented from engaging recess 254, the likelihood of the protuberances 234a, 234b contacting the portal is reduced, if not eliminated. Latches 232a, 232b prevent forward movement of knife 205 until surgical stapling apparatuses 100, 200 are loaded with unused cartridge assembly.

In accordance with the instant disclosure, if flange 218b should come out of contact with body portions 240a, 240b, the biasing force provided springs 267 against arm portions 245a, 245b would cause protuberances 234a, 234b and/or trailing surfaces 260a, 260b to engage recess 254 and extend a predetermined distance therethrough to engage flange 218b and, thus, prevent knife 205 from traveling distally therepast (Fig. 35).

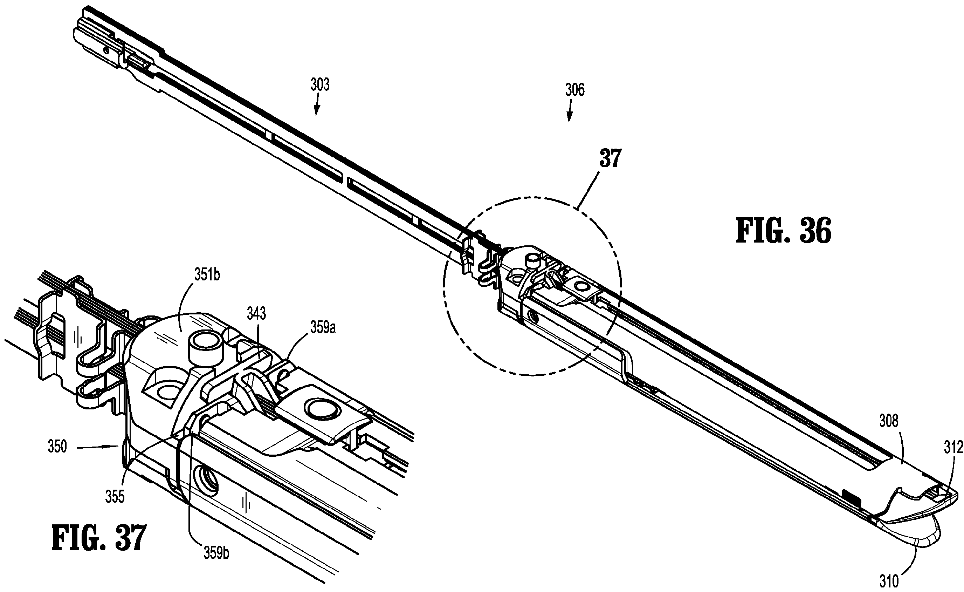

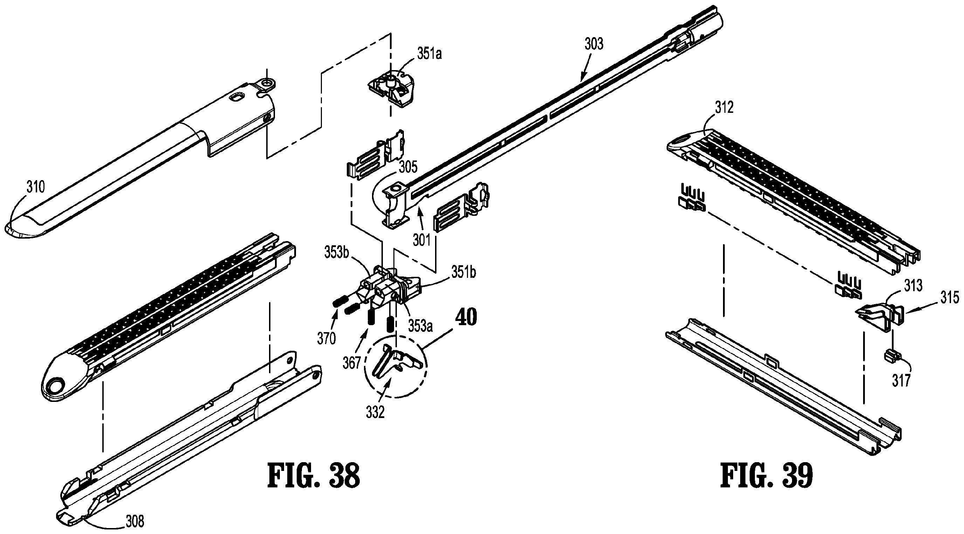

With reference to Figs. 36-47, an example reload 306 includes a locking mechanism according to another embodiment of the instant disclosure and is configured for use with surgical stapling apparatuses 100, 200 (Figs. 1 and 2).

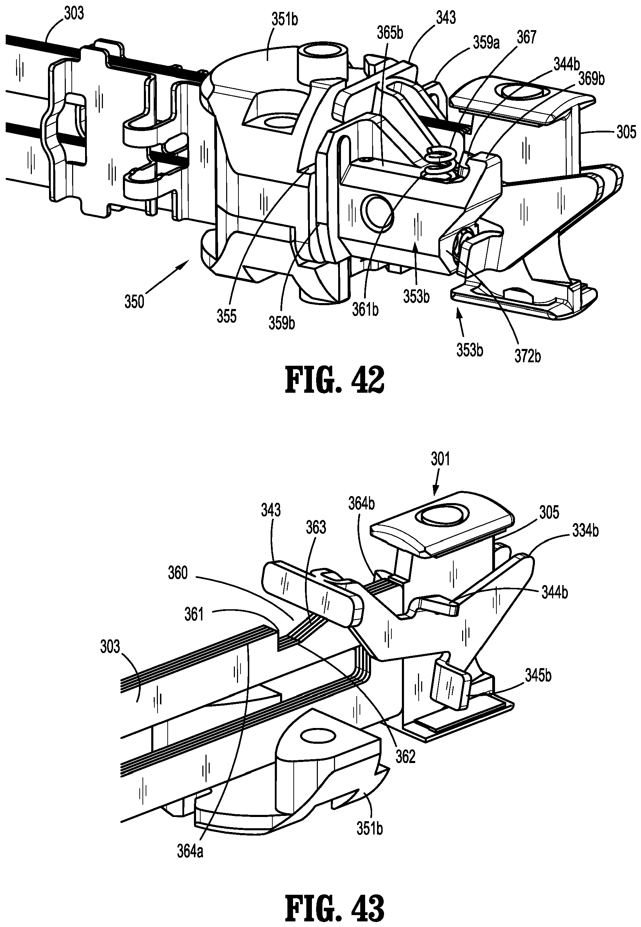

With initial reference to Figs. 37 and 42, a lower portion 351b of pivot assembly 350 includes two spaced-apart upright extensions 359a, 359b that are provided adjacent a shelf 355 to form a holding area for a locking member 343. Specifically, extensions 359a, 359b are positioned distally with respect to shelf 355 and extend a predetermined distance above shelf 355 to engage locking member 343 to prevent locking member 343 from moving distally past extensions 359a, 359b. Extensions 359a, 359b are spaced apart a predetermined distance from one another so as to allow a working end 301 to advance through a firing sequence of the surgical stapling apparatus 100, 200.

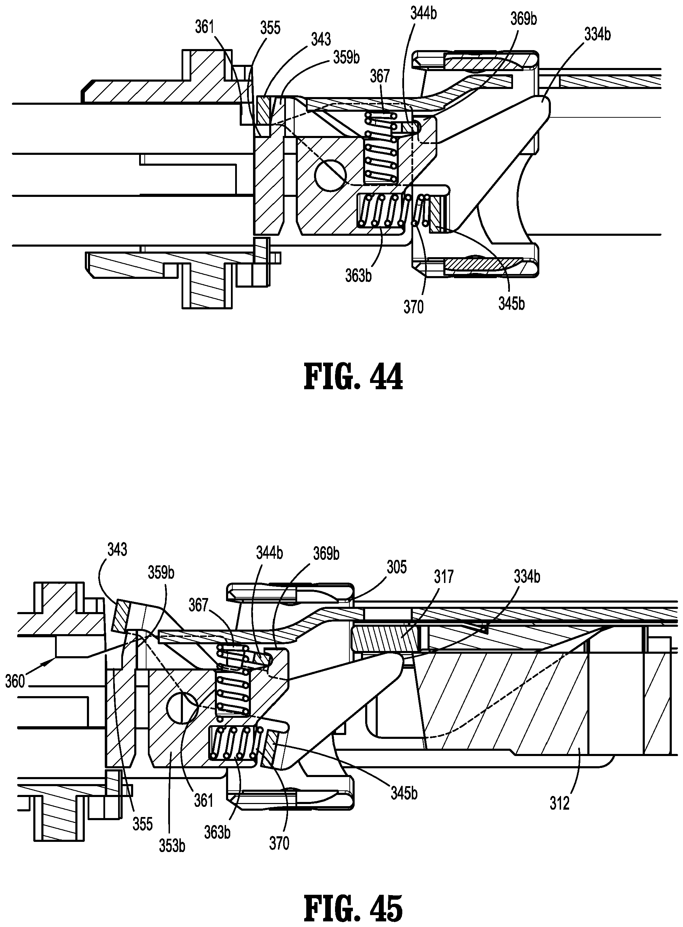

Continuing with reference to Fig. 42, a leg member 353b extends from extension 359b and includes a generally flat top surface 365b defining a cavity 361b (see Fig. 45) that is configured to house an optional spring 367, e.g., a compression spring. A claw portion 369b extends in a generally upright orientation from top surface 365b and is configured to couple to a corresponding hinge member 344b of latch 332 (Figs. 42 and 44) so as to allow hinge member 344b to pivot thereabout to facilitate sliding of locking member 343 along a notch 360 and/or a distal top surface 364b of drive beam members 303 (Fig. 43). A distal face 372b (Fig. 42) of leg member 353b defines a cavity 363b that is configured to house a spring 370, e.g., a compression spring, (Figs. 44-45). Spring 370 includes a predetermined spring constant and is configured to contact a lateral extension 345b of latch 332 to bias latch 332 in a generally upright configuration.

Extension 359a is identical to extension 359b and includes all the aforementioned components described with respect to extension 359b. Accordingly, a detailed description of extension 359a is not provided.

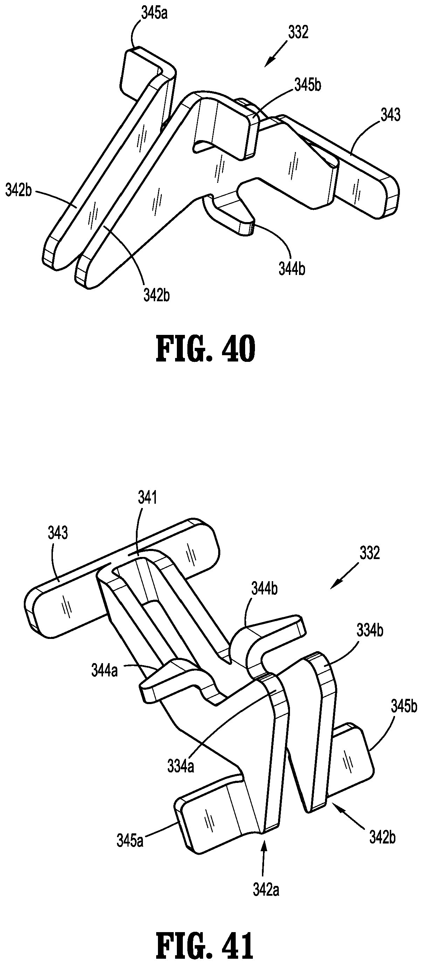

Referring now to Figs. 40-41, latch 332 is illustrated. Unlike latch 232, latch 332 is a single component having locking member 343 formed at a proximal end 341 and a bifurcated configuration including two (2) generally elongated members 342a, 342b extending distally therefrom. Members 342a, 342b are spaced apart a predetermined distance from one another to the working end 301 of the drive member to move therebetween during a firing sequence of surgical apparatuses 100, 200.

Hinge members 344a, 344b are provided at a medial portion of respective members 342a, 342b and include a generally arcuate configuration. Each of hinge members 344a, 344b extends a predetermined distance orthogonally from members 342a, 342b and curve outward therefrom to pivotably engage corresponding claw portions 369a, 369b to allow latch 332 to pivot as locking member 343 slides along drive beam members 303.

A pair of protuberances 334a, 334b are provided at a distal end of latch 332 and are configured to contact blocking member 317 (Fig. 45) when cartridge 312 is coupled to jaw member 308. Specifically, when protuberances 334a, 334b contact blocking member 317, latch 332 pivots about hinge members 344a, 344b which raises locking member 343 a predetermined distance and out of engagement with notch 360, as will be described in greater detail below.

Lateral extensions 345a, 345b are positioned proximally with respect to protuberances 334a, 334b and, when coupled to pivot assembly 350, adjacent coil sprigs 370 for contact therewith to urge protuberances 344a, 344b in a generally upward direction. Lateral extension 345b is configured to maintain coil spring 370 within cavity 363b as latch 332 pivots (Figs. 44-45). Likewise, lateral extension 345a is configured to maintain coil spring 370 within the corresponding cavity (not explicitly shown) as latch 332 pivots.

Fig. 43 illustrates a distal end of drive beam members 303. Unlike drive beam members 103, drive beam members 303 collectively define notch 360. Specifically, notch 360 is provided adjacent to where a distal end of the drive beam members 303 couple to knife 305, as best seen in Fig. 43. Notch 360 may be formed during manufacture of drive beam members 303 by suitable methods including but not limited to etching, stamping, cutting, etc. Notch 360 includes a generally upright proximal wall 361 that extends from a generally flat medial portion 362. Wall 361 extends upwardly to meet with a proximal top surface 364a of drive beam members 303 and is configured to selectively engage locking bar 343 of a latch 332 to lock-out knife 305 so as to prevent misfiring of knife 305, as described in greater detail below. A ramp portion 363 extends distally from medial portion 362 and is provided towards a distal end of notch 360. Ramp portion 363 may extend at any suitable angle distally from medial portion 362 and is configured to slidably engage locking bar 343 when knife 305 is translated proximally and distally. Ramp portion 363 extends distally to meet a distal top surface 364b of drive beam members 303. Distal top surface 364b is configured to allow locking bar 343 to slide thereon when knife 305 is moved to a retracted configuration.

Operation of surgical stapling apparatuses 100, 200 that utilize reload 306 is described herein. Initially, jaw members 308, 310 may be in an open configuration to load cartridge 312 onto jaw member 308 (Fig. 44). In accordance with the embodiment illustrated in Figs. 36-47, when cartridge 312 is not coupled to jaw member 308 the working end 301 of the drive member is locked out. Specifically, coil springs 370 contact lateral extensions 345a, 345b (in Fig. 44, only coil spring 370 is illustrated contacting extension 345b) to urge protuberances 343a, 343b in the generally upwardly direction, and locking member 343 in the generally downwardly direction into notch 360 and into contact with proximal wall 361. This contact between proximal wall 361 and locking member 343 maintains the working end 301 in a locked-out configuration.

Thereafter, cartridge 312 may be loaded onto jaw member 308. In the loaded configuration, blocking member 317 is positioned to contact with protuberances 334a, 334b. This contact between protuberances 334a, 334b and blocking member 317 forces protuberances 334a, 334b in a generally downwardly direction and causes latch 332 to pivot about pivot member 344a, 344b, which, in turn, causes locking member 343 to pivot in a generally upwardly direction and out of contact with proximal wall 361, see Fig. 45; with locking member 343 in this configuration, knife 305 may be fired.

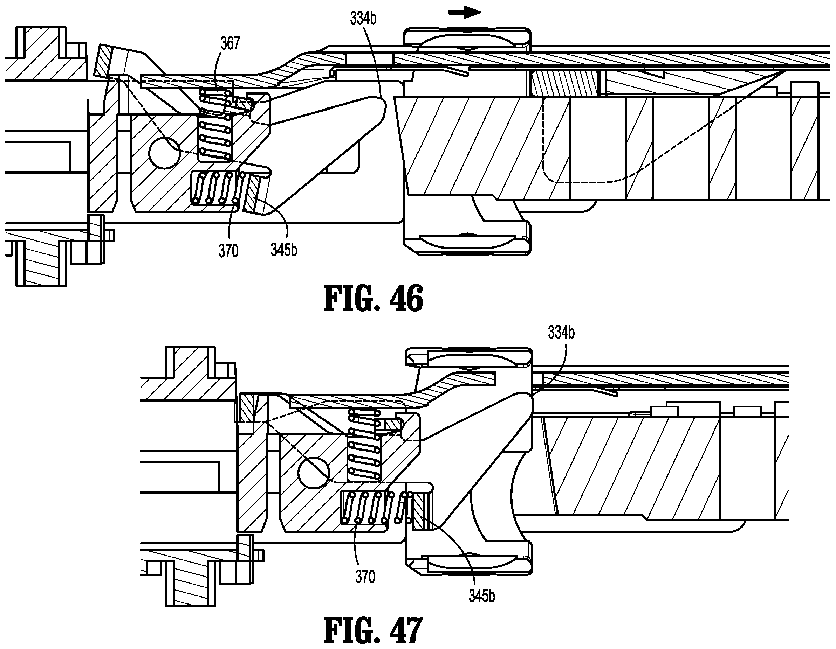

When working end 301 is advanced to staple and sever tissue, blocking member 317 moves distally with actuation sled 315 and out of contact with protuberances 334a, 334b (Fig. 46). Accordingly, protuberances 334a, 334b as a result of bias of spring 370 are once again forced in a generally upwardly direction and locking member 343 in the generally downwardly direction.

Subsequent to stapling and severing tissue, the working end 301 may be moved proximally and returned to its fully retracted configuration. As the working end 301 is being moved proximally, locking member 343 slides a predetermined distance along proximal top surface 364a until such time locking member 343 is forced downwardly into notch 360 and into contact with proximal wall 361. With locking member 343 engaged with notch 360, knife 305 is locked out and prevented from misfiring.

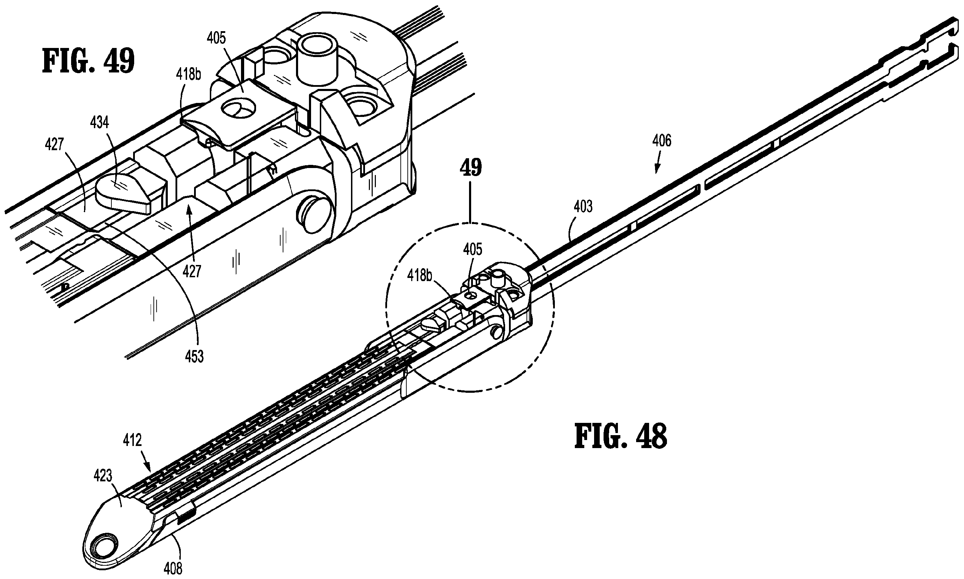

With reference to Figs. 48-59, a reload 406 according to the present invention includes a locking mechanism according to an embodiment of the instant disclosure and is configured for use with surgical stapling apparatuses 100, 200 is illustrated. Loading unit 406 can generally be configured as described above.

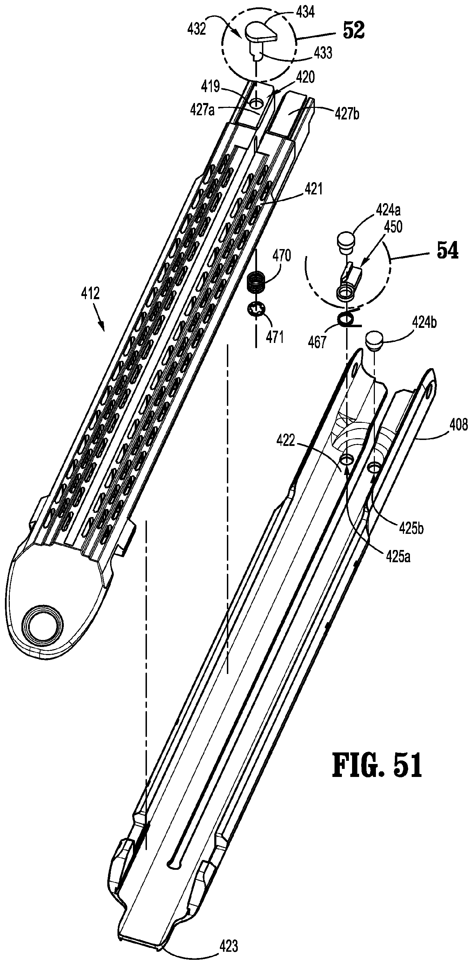

Beginning with reference to Figs. 48-51, reload 406 includes a cartridge 412 that is similar to the previously described cartridge assemblies, e.g., cartridge 112. Unlike cartridge 112, however, one or more recessed platform areas 427a, 427b are provided adjacent to a proximal end of tissue contacting surface 421 of cartridge 412, as best seen in Fig. 51.

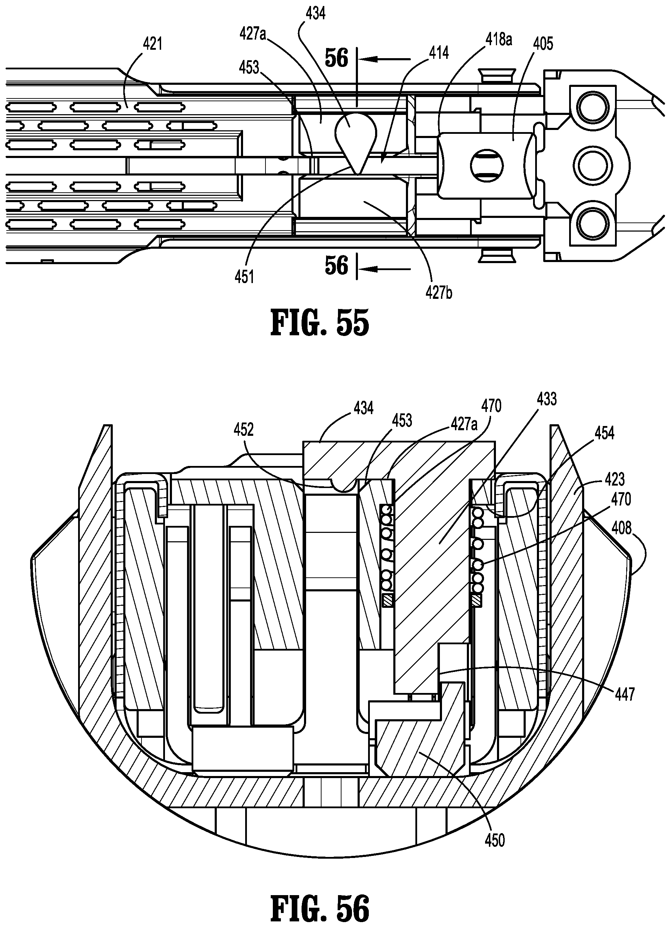

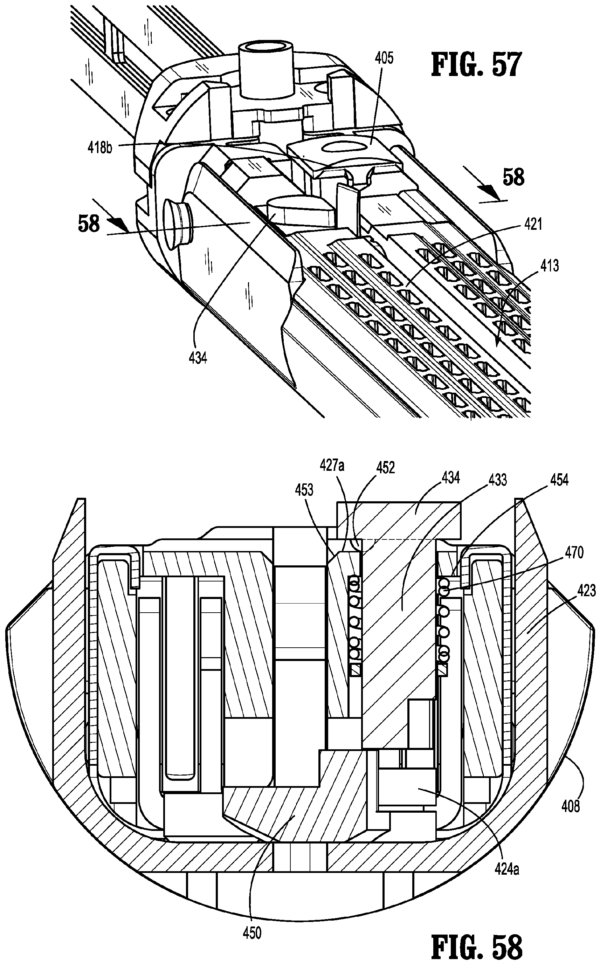

An aperture 420 is defined through platform area 427a and is configured to receive a post 433 of an actuator 432 (Fig. 51). Aperture 420 is configured to allow rotation of post 433 and a head portion 434 of actuator 432 when head portion 434 is contacted by a top flange 418b disposed on knife 405 (Figs. 48-49). In a pre-fired configuration (e.g., prior to top flange 418b contacting head portion 434), head portion 434 rests on platform area 427a (Fig. 56). In a post-fired configuration (e.g., subsequent to top flange 418b contacting head portion 434), head portion 434 is raised a predetermined distance above platform area 427a (Fig. 58).

A pair of apertures 425a, 425b of suitable configuration are defined through a bottom interior wall 422 of cartridge housing 423 and are configured to receive a corresponding rivet 424a, 424b therein (Fig. 51). Aperture 425a is in vertical registration with aperture 420 to align post 433 with an interlock 450 (see Figs. 51 and 56).

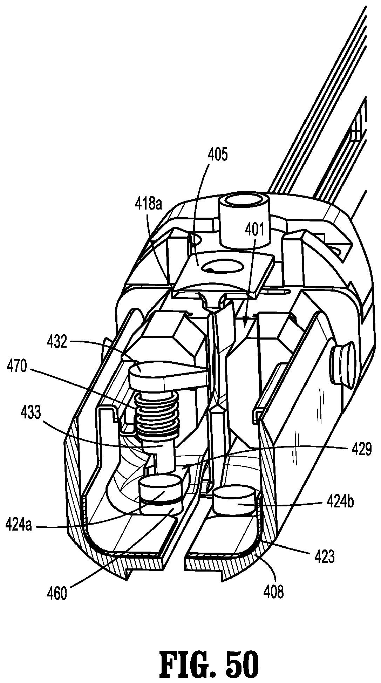

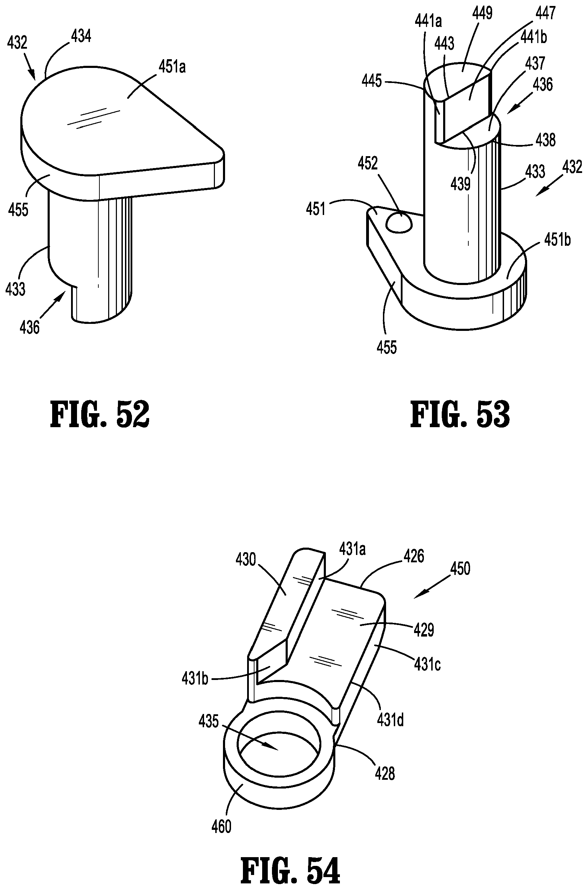

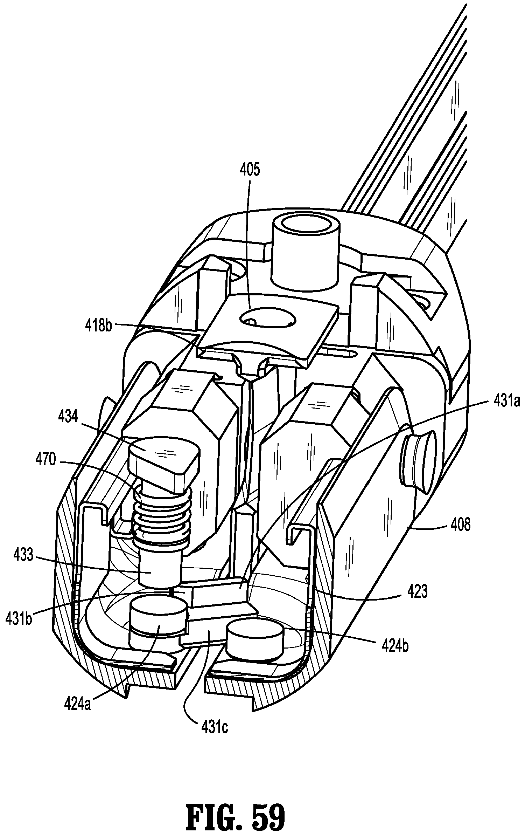

With reference to Figs. 52-53, actuator 432 is illustrated. Actuator 432 is rotatable within aperture 420 from an initial configuration wherein head portion 434 rests on platform 427a and post 433 is engaged with interlock 450 (Fig. 56) to a final configuration wherein head portion 434 is raised above platform 427a and post 433 is disengaged from interlock 450 (Fig. 58). When post 433 is engaged with interlock 450, working end 401 is free to move distally (Figs. 50 and 56). Conversely, when post 433 is disengaged from interlock 450, the working end 401 is locked out and unable to move distally (Figs. 58 and 59).

Continuing with reference to Figs. 52-53, post 433 extends from head portion 434 and includes a generally elongated, cylindrical configuration. A notch 436 is provided adjacent a bottom portion of post 433 and is defined by a generally hemispherical top surface 437 that is defined by a semi-circular peripheral edge and an interior linear edge 439. Edge 439 meets a wall 447 of suitable configuration that extends in a generally orthogonal direction from top surface 437 to meet an interior linear edge 443 that meets with a semi-circular peripheral edge 445. Linear edge 443 and peripheral edge 445 define a generally hemispherical bottom surface 449. A pair of beveled side edges 441 a, 441 b are provided on wall 447 and extend between bottom and top surfaces 449 and 437, respectively, to facilitate rotation of post 433 about interlock 450.

Head portion 434 includes top and bottom surfaces 451a, 451b that are joined by a sidewall 455 extending in a curvilinear manner around top and bottom surfaces 451a, 451b to form a generally cone-like configuration (Figs. 52-53). A tip 451 of head portion 434 is configured to extend at least partially within a knife channel 414 when actuator 432 and a working end 401 are in the pre-fired configuration, see Fig. 55 for example. A protuberance 452 is provided on bottom surface 451b (Fig, 53) and is configured to contact an interior edge 453 (Fig. 55) that extends into knife channel 414. Protuberance 452 may include any suitable configuration. In the illustrated embodiment, for example, protuberance 452 includes a generally rounded configuration, e.g., a dot-like configuration. The rounded configuration of protuberance 452 facilitates raising head portion 434 above platform area 427a when protuberance 452 contacts an interior edge 453 disposed adjacent platform area 427a (Fig. 56). In addition, interior edge 453 may be beveled/slanted (Figs. 56 and 58) or otherwise configured to facilitate raising head portion 434 above platform 427a when protuberance contacts interior edge 453.

A spring 470 (e.g., a coil spring or other suitable resilient member (Figs. 50-51) is operably coupled to post 433 and is configured to bias actuator 432 towards the bottom interor wall 422 of the cartridge assembly 412 as shown in Figs. 56 and 58. Specifically, spring 470 is configured to contact an interior wall 454 that lies beneath tissue contacting surface 421 of cartridge 412 (Figs. 56 and 58). One or more suitable coupling methods and/or devices may be utilized to couple spring 470 to post 433. In the illustrated embodiment, for example, a lock washer 471 is utilized to couple spring 470 to post 433 (Fig. 51). Lock washer 471 is also utilized to rotatably secure post 433 of actuator 432 within aperture 420.

Referring to Fig. 54, interlock 450 is illustrated. Interlock 450 is rotatable within aperture 425a from an initial configuration wherein interlock 450 is engaged with sidewall 447 of post 433 (Fig. 56) to a final configuration wherein interlock 450 is disengaged from sidewall 447 (Fig. 58) and engaged with rivet 424b (Fig. 59). When interlock 450 is engaged with sidewall 447, interlock 450 is positioned outside of a translation path of the working end 401 and the working end 401 is free to move distally (Figs. 50 and 56). Conversely, when interlock 450 is disengaged from sidewall 447, interlock 450 is positioned inside of translation path of the working end 401 and the working end 401 is locked out and unable to move distally (see Figs. 58 and 59 for example).

Continuing with reference to Fig. 54, interlock 450 includes a stepped configuration having a proximal end 426 and a distal end 428. Proximal end 426 includes a generally rectangular configuration having a relatively flat top surface 429 that is configured to receive bottom surface 449 of post 433 thereon (Fig. 50). A bottom surface (not explicitly shown) of interlock 450 is configured to slide along bottom interior wall 422 as interlock 450 rotates. A sidewall 431c extends from the bottom surface and meets top surface 429 forming an edge 431d. Sidewall 431c forms a first step and is configured to contact rivet 424b when interlock is in the post-fired configuration (Fig. 59). A generally rectangular upright extension 430 of suitable configuration is provided on top surface 429 and includes interior sidewall portions 431a, 431b that form second step. Sidewall portion 431a extends in a straight manner a predetermined distance from a proximal edge of top surface 429. Sidewall portion 431b extends at an angle a predetermined distance from a distal end of sidewall portion 431a. In the pre-fired configuration, sidewall 447 of post 433 is flush with sidewall portion 431a (Fig. 50). As post 433 rotates during a firing sequence, the beveled configuration of side edges 441a, 441b in conjunction with the angle at which sidewall portion 431b extends facilitates the transition of post 433 and interlock 450 from their pre-fired configuration to their post-fired configuration.

A generally circumferential sidewall 460 (Fig. 54) is provided at distal end 428 and includes an aperture 435 of suitable configuration defined therethrough. Aperture 435 is configured to receive rivet 424a therein for coupling interlock 450 to cartridge housing 423. Rivet 424a is configured to allow rotation of interlock 450 from the pre-fired configuration to the post-fired configuration (see Figs. 50-51 and 59).

A spring 467, e.g., a torsion spring 467, having a suitable spring coefficient operably couples via one or more suitable coupling methods and/or devices to the bottom surface of interlock 450. (Fig. 51). Spring 467 is configured to bias interlock 450 towards the post-fired configuration, as best seen in Fig. 59. In the post-fired configuration, sidewall 431c engages rivet 424b to prevent rotation of interlock 450 past a predetermined point and lockout the working end 401 (Fig. 59).

In use, actuator 432 is, initially, in the pre-fired configuration with tip 451 in the translation path of the working end 401 (Figs. 50 and 55-56). Thereafter, the working end 401 may be advanced and flange 418a contacts head portion 434 of actuator 432, which, in turn, causes post 433 to rotate and protuberance 452 to ride up along interior edge 453 and onto platform 427a. As post 433 rotates, sidewall 447 rotates about sidewall 431a and begins to rise above extension 430 as a result of the upward bias of spring 470.

Once protuberance 452 is moved into position on platform 427a, sidewall 447 will be sufficiently raised so as to disengage sidewall 431 a (Figs. 57 -79). As a result thereof, interlock 450 under the bias of spring 467 is forced to rotate until such time that sidewall 431c contacts rivet 424b (Figs. 58-59).

The working end 401 may be moved back to its retracted, pre-fired configuration against the biasing force of spring 467. Specifically, a trailing surface (not explicitly shown, see trailing surface 118d in Fig. 3B for example) of the working end 401 contacts sidewall 431c so as to push interlock 450 proximally and out of engagement with rivet 424b until the working end 401 is moved therepast and to its retracted, pre-fired configuration. The trailing surface is desirably a cam surface or angled to facilitate this. Once the working end 401 is moved back to its retracted, pre-fired configuration, interlock 450 is once again forced forward by spring 467 and into contact with rivet 424b (Fig. 59). With interlock 450 in contact with rivet 424b, the working end 401 is locked out and prevented from distal translation.

The figures show a replaceable loading unit with surgical stapling jaws that has a shaft (such as a shaft 109) that can be attached to a surgical stapling apparatus. Other configurations are contemplated. For example, the replaceable loading unit can itself have a removable and replaceable cartridge assembly. Alternatively, the jaws of the instrument can be permanently attached and configured to receive a removable and replaceable cartridge.

In any of the embodiments disclosed herein, the instrument housing 102 can be manually operated or powered.

While several embodiments of the disclosure have been shown in the drawings, it is not intended that the disclosure be limited thereto. Therefore, the above description should not be construed as limiting, but merely as exemplifications of particular embodiments. Those skilled in the art will envision other modifications within the scope of the claims appended hereto.

## Claims

1. A surgical stapling apparatus (100, 200), comprising a reload (406), the reload comprising:
a first jaw member (108, 408);
a second jaw member (110);
a cartridge (412) configured to be selectively coupled to the first jaw member, the cartridge defining a knife channel (414) and including a housing (423), a plurality of fasteners (117a), a tissue contacting surface (421), and one or more recessed platform areas (427a, 427b) adjacent to a proximal end of the tissue contacting surface (421), wherein an aperture (420) is defined through one platform area (427a);
a drive member comprising a drive beam (403) having a working end (401) comprising a top flange (418a), the drive member being translatable from a retracted position to an advanced position;
an actuator (432) comprising a head portion (434) and a post (433) extending from the head portion (434), the head portion (434) comprising a tip (451), a bottom surface (451b), and a protuberance (452) provided on the bottom surface (451b), wherein the post (433) is received within the aperture (420), and the aperture is configured to allow rotation of the post (433) and head portion (434);
a spring (470) operably coupled to the post (433) and configured to bias the actuator (432) in a direction towards the bottom interior wall (422) of the cartridge assembly (412); and
an interlock (450) comprising an extension (430) rotatably coupled to the cartridge housing (423) and biased into the translation path of the working end (401) by a biasing member (467),
wherein the working end (401), the actuator (432), and the interlock (450) each define a pre-fired configuration and a post-fired configuration;
wherein in their pre-fired configuration, the working end (401) is in the retracted position, the tip (451) of the actuator (432) extends into the translation path of the working end (401), the post (433) is engaged with the interlock (450) against the bias of the biasing member (467), and the interlock is positioned outside of the translation path of the working end (401);
wherein in their post-fired configuration, the working end (401) is in the advanced position, the tip (451) of the actuator (432) is rotated out of the translation path of the working end (401), the post (433) is disengaged from the interlock (450), and the interlock (450) is positioned inside the translation path of the working end (401);
wherein during firing, the working end (401) is advanced and the top flange (418a) contacts the head portion (434) of the actuator (432), causing the post (433) to rotate and the protuberance (452) to ride up along an interior edge (453) of the platform (427a) and onto the platform (427a), causing the post (433) to rise above the extension (430) and disengage the interlock (450) which is consequentially forced by the biasing member (467) to rotate into the translation path of the working end (401);
wherein after firing, the working end (401) is movable back to the retracted position, and
wherein after the working end (401) is moved back to the retracted position after firing, the working end (401) is prevented from distal translation by the interlock (450).

2. The surgical stapling apparatus according to claim 1, wherein the drive member includes a knife (405) configured to sever tissue captured between the first and second jaw members as the drive member is advanced towards the advanced position.

3. The surgical apparatus according to any preceding claim, wherein the head portion (434) has a cone-like shape.

4. The surgical apparatus according to any preceding claim, wherein the drive member includes a trailing surface (118d), the trailing surface engaging the interlock (450) when the drive member is moved from the advanced position to the retracted position.

5. The surgical apparatus according to any preceding claim, further comprising one or more motors.

6. The surgical apparatus according to any one of claims 1-4, further comprising a manually operated trigger.

## Patentansprüche

1. Chirurgisches Klammergerät (100, 200), umfassend eine Nachladung (406), wobei die Nachladung umfasst:
ein erstes Backenglied (108, 408);
ein zweites Backenglied (110);
eine Patrone (412), die konfiguriert ist, um selektiv an das erste Backenglied gekoppelt zu werden, wobei die Patrone einen Messerkanal (414) definiert und ein Gehäuse (423), eine Vielzahl von Heftmitteln (117a), eine Gewebekontaktfläche (421) und einen oder mehrere vertiefte Plattformbereiche (427a, 427b) angrenzend an ein proximales Ende der Gewebekontaktfläche (421) aufweist, wobei eine Öffnung (420) durch einen Plattformbereich (427a) definiert ist;
ein Antriebsglied, das einen Antriebsbalken (403) mit einem Arbeitsende (401) umfasst, das einen oberen Flansch (418a) umfasst, wobei das Antriebsglied von einer eingezogenen Position zu einer vorgeschobenen Position versetzbar ist;
einen Stellantrieb (432), der einen Kopf-Bereich (434) und einen sich von dem Kopf-Bereich (434) erstreckenden hinteren Bereich (433) umfasst, wobei der Kopf-Bereich (434) eine Spitze (451), eine Bodenfläche (451b) und einen an der Bodenfläche (451b) vorgesehenen Vorsprung (452) umfasst, wobei der hintere Bereich (433) innerhalb der Öffnung (420) aufgenommen ist und die Öffnung konfiguriert ist, um eine Drehung des hinteren Bereichs (433) und des Kopf-Bereichs (434) zu ermöglichen;
eine Feder (470), die betriebsfähig mit dem hinteren Bereich (433) gekoppelt ist und konfiguriert ist, um den Stellantrieb (432) nach unten in Richtung der unteren Innenwand (422) der Patronenanordnung (412) zu forcieren; und
eine Verriegelung (450), die eine Verlängerung (430) umfasst, die drehbar mit dem Patronengehäuse (423) gekoppelt ist und durch ein Rückstellelement (467) in den Versetzungspfad des Arbeitsendes (401) forciert ist,
wobei das Arbeitsende (401), der Stellantrieb (432) und die Verriegelung (450) jeweils eine Vor-Feuer-Konfiguration und eine Nach-Feuer-Konfiguration definieren;
wobei in ihrer Vor-Feuer-Konfiguration sich das Arbeitsende (401) in der eingezogenen Position befindet, sich die Spitze (451) des Stellantriebs (432) in den Versetzungspfad des Arbeitsendes (401) erstreckt, der hintere Bereich (433) gegen die Rückstellung des Rückstellelements (467) in die Verriegelung (450) eingreift und die Verriegelung außerhalb des Versetzungspfads des Arbeitsendes (401) positioniert ist;
wobei in ihrer Nach-Feuer-Konfiguration sich das Arbeitsende (401) in der vorgeschobenen Position befindet, die Spitze (451) des Stellantriebs (432) aus dem Versetzungspfad des Arbeitsendes (401) herausgedreht ist, der hintere Bereich (433) aus der Verriegelung (450) ausgeklinkt ist und die Verriegelung (450) innerhalb des Versetzungspfads des Arbeitsendes (401) positioniert ist;
wobei während des Feuerns das Arbeitsende (401) vorgeschoben ist und der obere Flansch (418a) mit dem Kopf-Bereich (434) des Stellantriebs (432) in Kontakt steht, wodurch bewirkt wird, dass sich der hintere Bereich (433) dreht und der Vorsprung (452) entlang einer Innenkante (453) der Plattform (427a) und auf die Plattform (427a) aufsteigt, wodurch bewirkt wird, dass sich der hintere Bereich (433) über der Verlängerung (430) erhebt und die Verriegelung (450) ausklinkt, die folglich von dem Rückstellelement (467) gezwungen wird, sich in den Versetzungspfad des Arbeitsendes (401) zu drehen;
wobei nach dem Feuern das Arbeitsende (401) zurück zu der eingezogenen Position beweglich ist, und
wobei nachdem das Arbeitsende (401) nach dem Feuern zurück zu der eingezogenen Position bewegt wurde, das Arbeitsende (401) durch die Verriegelung (450) an einer distalen Versetzung gehindert wird.

2. Chirurgisches Klammergerät nach Anspruch 1, worin das Antriebsglied ein Messer (405) aufweist, das konfiguriert ist, um das Gewebe zu durchtrennen, das zwischen den ersten und zweiten Backengliedern ergriffen wird, wenn das Antriebsglied in Richtung der vorgeschobenen Position vorgeschoben wird.

3. Chirurgisches Gerät nach einem vorstehenden Anspruch, worin der Kopf-Bereich (434) eine konusartige Form aufweist.

4. Chirurgisches Gerät nach einem vorstehenden Anspruch, worin das Antriebsglied eine Schleppfläche (118d) aufweist, wobei die Schleppfläche in die Verriegelung (450) eingreift, wenn das Antriebsglied von der vorgeschobenen Position zu der eingezogenen Position bewegt wird.

5. Chirurgisches Gerät nach einem vorstehenden Anspruch, welches zusätzlich einen oder mehrere Motoren umfasst.

6. Chirurgisches Gerät nach einem der Ansprüche 1-4, welches zusätzlich einen von Hand bedienten Auslöser umfasst.

## Revendications

1. Appareil d'agrafage chirurgical (100, 200), comprenant une recharge (406), la recharge (406) comprenant :
un premier élément formant mâchoire (108, 408) ;
un second élément formant mâchoire (110) ;
une cartouche (412) configurée pour s'accoupler de manière sélective au premier élément formant mâchoire, la cartouche définissant un canal pour couteau (414) et incluant un boîtier (423), une pluralité d'attaches (117a), une surface en contact avec le tissu (421) et une ou plusieurs zones de plateforme en creux (427a, 427b) adjacentes à une extrémité proximale de la surface en contact avec le tissu (421), dans lequel une ouverture (420) est définie à travers une zone de plateforme (427a) ;
un élément d'entraînement comprenant une poutre d'entraînement (403) ayant une extrémité de travail (401) comprenant une bride supérieure (418a), l'élément d'entraînement pouvant effectuer une translation d'une position rétractée à une position avancée ;
un dispositif d'actionnement (432) comprenant une partie de tête (434) et un tenon (433) s'étendant de la partie de tête (434), la partie de tête (434) comprenant un embout (451), une surface inférieure (451b) et une protubérance (452) ménagée sur la surface inférieure (451b), dans lequel le tenon (433) est reçu dans l'ouverture (420), et l'ouverture est configurée pour permettre la rotation du tenon (433) et de la partie de tête (434) ;
un ressort (470) couplé en service au tenon (433) et configuré pour presser le dispositif d'actionnement (432) en direction de la paroi interne inférieure (422) de l'ensemble de cartouche (412) ; et
un verrou (450) comprenant une extension (430) couplée en rotation au boîtier de cartouche (423) et pressée dans le trajet de translation de l'extrémité de travail (401) par un élément de sollicitation (467),
dans lequel l'extrémité de travail (401), le dispositif d'actionnement (432) et le verrou (450) définissent chacun une configuration avant tir et une configuration après tir ;
dans lequel, dans leur configuration avant tir, l'extrémité de travail (401) est en position rétractée, l'embout (451) du dispositif d'actionnement (432) s'étend dans le trajet de translation de l'extrémité de travail (401), le tenon (433) est en prise avec le verrou (450) contre la sollicitation de l'élément de sollicitation (467) et le verrou est positionné à l'extérieur du trajet de translation de l'extrémité de travail (401) ;
dans lequel, dans leur position après tir, l'extrémité de travail (401) est en position avancée, l'embout (451) du dispositif d'actionnement (432) est soumis à une rotation hors du trajet de translation de l'extrémité de travail (401), le tenon (433) est dégagé du verrou (450) et le verrou (450) est positionné à l'intérieur du trajet de translation de l'extrémité de travail (401) ;
dans lequel, au cours du tir, l'extrémité de travail (401) est avancée et la bride supérieure (418a) vient en contact avec la partie de tête (434) du dispositif d'actionnement (432), amenant le tenon (433) à tourner et la protubérance (452) à s'élever le long d'un bord interne (453) de la plateforme (427a) et sur la plateforme (427a), amenant le tenon (433) à s'élever au-dessus de l'extension (430) et à dégager le verrou (450) qui est par suite forcé par l'élément de sollicitation (467) à tourner dans le trajet de translation de l'extrémité de travail (401) ;
dans lequel, après le tir, l'extrémité de travail (401) peut être ramenée à la position rétractée et
dans lequel, une fois que l'extrémité de travail (401) est ramenée à la position rétractée après le tir, l'extrémité de travail (401) est empêchée d'effectuer une translation distale par le verrou (450).

2. Appareil d'agrafage chirurgical selon la revendication 1, dans lequel l'élément d'entraînement comprend un couteau (405) configuré pour sectionner le tissu capturé entre les premier et second éléments de mors lorsque l'élément d'entraînement est avancé vers la position avancée.

3. Appareil chirurgical selon l'une quelconque des revendications précédentes, dans lequel la partie de tête (434) a une forme conique.

4. Appareil chirurgical selon l'une quelconque des revendications précédentes, dans lequel l'élément d'entraînement comprend une surface de fuite (118d), la surface de fuite venant en prise avec le verrou (450) lorsque l'élément d'entraînement est déplacé de la position avancée à la position rétractée.

5. Appareil chirurgical selon l'une quelconque des revendications précédentes, comprenant en outre un ou plusieurs moteurs.

6. Appareil chirurgical selon l'une quelconque des revendications 1 à 4, comprenant en outre un déclencheur à commande manuelle.
